# EUROPÄISCHE PATENTSCHRIFT

(11) **EP 2 036 926 B1**
(45) Veröffentlichungstag und Bekanntmachung des Hinweises auf die Patenterteilung: **14.08.2013**
(21) Anmeldenummer: 08019619.9
(22) Anmeldetag: 08.04.1999
(51) Int. Cl.: C07K 16/46, C07K 16/00, C12N 15/13, C07K 19/00, C12N 15/62, C12N 15/70, C12N 1/21, C12N 15/85, C12N 5/10, A61K 39/395

(54) **Einzelkettiges, mehrfach-antigenbindendes Molekül, dessen Herstellung und Verwendung**
Single-chain, multiple antigen forming molecule, its manufacture and use
Molécule à une chaîne, à liaison multiple d'antigène, sa fabrication et son utilisation

(30) Priorität: 09.04.1998 DE 19816141; 18.06.1998 DE 19827239
(43) Veröffentlichungstag der Anmeldung: 18.03.2009
(62) Teilanmeldung aus: 99106176.3
(73) Patentinhaber: Affitech Research AS, 0349 Oslo (NO)
(72) Erfinder: Kontermann, Roland, 35085 Ebsdorfergrund (DE); Sedlacek, Hans-Harald, 35041 Marburg (DE); Müller, Rolf, 35037 Marburg (DE)
(74) Vertreter: Zwicker, Jörk

(56) Entgegenhaltungen:
- EP-A2- 0 610 046
- WO-A1-93/11161
- M. GRUBER ET AL.: "Efficient tumor cell lysis mediated by a bispecific single chain antibody expressed in Escherichia coli." THE JOURNAL OF IMMUNOLOGY, Bd. 152, Nr. 11, 1. Juni 1994 (1994-06-01) , Seiten 5368-5374, XP000872832 Baltimore, MD, VSA
- K. FITZGERALD ET AL.: "Improved tumour targeting by disulphide stabilized diabodies expressed in Pichia pastoris." PROTEIN ENGINEERING, Bd. 10, Nr. 10, Oktober 1997 (1997-10), Seiten 1221-1225, XP002144896 Oxford, GB
- Z. ZHU ET AL.: "Remodeling domain interfaces to enhance heterodimer formation." PROTEIN SCIENCE, Bd. 6, Nr. 4, April 1997 (1997-04), Seiten 781-788, XP000929847 New York, NY, VSA
- DATABASE MEDLINE [Online] US NATIONAL LIBRARY OF MEDICINE (NLM), BETHESDA, MD, US Juni 2004 KORN TINA ET AL: 'Recombinant bispecific antibodies for the targeting of adenoviruses to CEA-expressing tumour cells: a comparative analysis of bacterially expressed single-chain diabody and tandem scFv.' Database accession no. NLM15170735 & KORN TINA ET AL: "Recombinant bispecific antibodies for the targeting of adenoviruses to CEA-expressing tumour cells: a comparative analysis of bacterially expressed single-chain diabody and tandem scFv.", THE JOURNAL OF GENE MEDICINE JUN 2004 LNKD- PUBMED:15170735, vol. 6, no. 6, June 2004 (2004-06), pages 642-651, ISSN: 1099-498X
- Alberts et al.: "Molekularbiologie der Zelle, VCH. 3. Auflage", 1995 ISBN: 3-527-30055-4 page 1556,

## Beschreibung

Die vorliegende Erfindung betrifft ein einzelkettiges, mehrfach-antigenbindendes Molekül, mit verschiedenen variablen Domänen einer schweren bzw. einer leichten Kette eines Immunglobulins, die in Form eines VH-VL-Konstruktes verbunden sind, welche wiederum über ein Peptid miteinander verbunden sind, sowie deren Herstellung und Verwendung als Arzneimittel oder Diagnostikum.

Bispezifische Antikörper, die zwei verschiedene Antigene, z.B. ein Tumorzelloberflächenantigen und ein Effektormolekül erkennen, finden eine breite Anwendung in der experimentellen Immuntherapie (Fanger et al., Crit. Rev. Immunol. 12, 101-124 (1992); van de Winkel et al., Immunol. Today 18, 562-564 (1997)). Zu den Effektorfunktionen, die durch bispezifische Antikörper rekrutiert werden, zählen u.a. solche, die natürlicherweise im Organismus vorkommen, wie z.B. zytotoxische und phagozytierende Zellen, Komplementkomponenten, Zytokine und thrombolytische und fibrinolytische Enzyme als auch körperfremde Effektormoleküle, wie z.B. Toxine, Prodrug-konvertierende Enzyme und Radionuklide. So führte z.B. die Injektion bispezifischer Antikörper gegen das Hodgkin's-Tumor-assoziierte Antigen CD30 und die T-Zell-Antigene CD3 bzw. CD28 in xenotransplantierte Tumoren zur Rekrutierung und Stimulierung von zytotoxischen T-Zellen und zur Induktion einer tumoriziden Aktivität (Renner et al., Science 264, 833, 1994). In einem anderen Ansatz wurde ein bispezifischer Antikörper gegen CD30 und alkalische Phosphatase dazu benutzt, das Enzym an die Tumorstelle zu rekrutieren und damit die untoxische Vorstufe eines Wirkstoffes in einen toxischen Wirkstoff zu überführen (Sahin et al., Cancer Res. 50, 6944-6948, 1990).

Eine Alternative zur Injektion des gereinigten bispezifischen Antikörpers ist die Expression und Sekretion dieser bispezifischen Antikörper durch in vitro oder in vivo transfizierte Zellen. Der Vorteil dieser Strategie ist, daß die Produktion des bispezifischen Antikörpers von der transduzierten Zelle in vivo erfolgt und somit keine aufwendige Produktion und Reinigung des bispezifischen Antikörpers vor einer Injektion notwendig ist. Darüber hinaus kann durch Wahl geeigneter Expressionssysteme die Expression des bispezifischen Antikörpers lokal, in Organen oder einem Tumor oder auch systemisch gesteuert werden.

Bispezifische Antikörper können beispielsweise durch chemische Quervernetzung (Nisonoff et al., Nature 194, 355, 1962) hergestellt werden. Bei der chemischen Quervernetzung monoklonaler bzw. polyklonaler Antikörpermoleküle tierischen Ursprungs kann ein nicht geringer Anteil inaktiviert werden. Zusätzlich entstehen sowohl Hetero- als auch Homodimere. Homodimere müssen durch aufwendige Verfahren von den gewünschten Heterodimeren abgetrennt werden. Hybridomzellen, die bispezifische Antikörper produzieren, sogenannte Hybrid-Hybridome, lassen sich nur relativ aufwendig herstellen, da hier zwei verschiedene Hybridome miteinander fusioniert werden müssen (Milstein et al., Nature 305, 537, 1983). Der Anteil an funktionellen Heterodimeren ist relativ gering, theoretisch nur 10%, da die schweren und leichten Ketten der beiden Antikörpermoleküle beliebig miteinander assoziieren können. Als Ausgangsmaterial werden hauptsächlich murine monoklonale Antikörper verwendet, welche wiederum für den Menschen immunogen sind.

Verschiedene bivalente bzw. bispezifische Antikörpermoleküle können auch rekombinant hergestellt und in Bakterien bzw. eukaryotischen Zellen exprimiert werden (WO 93/06217). Allen rekombinanten Antikörpermolekülen ist gemein, daß für ihre Herstellung sowohl murine als auch humane Ausgangsmoleküle verwendet werden können. Unterschiedliche Methoden wurden entwickelt, um bispezifische rekombinante Antikörpermoleküle möglichst effizient herstellen zu können. Mit diesen Methoden lassen sich unterschiedliche Gruppen von Molekülen herstellen.

Bei einer Gruppe von Molekülen werden die variablen Teile der Antikörper mit konstanten Immunglobulindomänen (Fc, CH3, CL) fusioniert, um eine Dimerisierung zu erreichen (Hu et al., Cancer Res. 56, 3055-3061 (1994); Hayden et al., ther. Immunol. 1, 3-15 (1994)). Hierbei erfolgt jedoch keine Selektion für heterodimere Moleküle, so daß auf diese Weise überwiegend bivalente Homodimere entstehen. Die Expression dieser Moleküle in funktioneller Form ist zudem auf eukaryotische Zellen beschränkt.

Bei einer weiteren Gruppe von Molekülen werden die variablen Teile der Antikörper mit Peptiden bzw. Proteindomänen aus anderen Proteinen zur Herstellung von bivalenten bzw. bispezifischen Molekülen fusioniert (Plückthun und Pack, Immunotechnol. 3, 83-105 (1997)). Auch hier erfolgt in der Regel eine Bildung von Homo- wie auch Heterodimeren aufgrund der zufälligen Assoziation. Zusätzlich enthalten diese Moleküle einen z.T. erheblichen Anteil von Fremdsequenzen, so daß mit einer deutlichen Immunogenität zu rechnen ist.

Bei einer dritten Gruppe von Molekülen werden geringfügige Modifikationen von rekombinanten Fv-Fragmenten, in der Regel einzelkettige Fv-Fragmente (scFv), zur Herstellung von bivalenten bzw. bispezifischen Molekülen verwendet (Holliger und Winter, Curr. Opin. Biotechnol. 4, 446-449 (1993)). Hierzu zählen die Dimerisierung durch zusätzliche Cysteine am C-Terminus der scFv-Ketten (MacCartney et al., Protein Engin. 8, 301-314 (1994)). Hierbei entstehen jedoch sowohl Homo- als auch Heterodimere und bei der Expression in Bakterienzellen entstehen nicht funktionelle Aggregate. Tandem-scFv-Moleküle der Struktur scFv(A)-Linker-scFv(B) (Mallender und Voss, J. Biol. Chem. 269, 199-206, 1994) lassen sich sowohl in Bakterien als auch in eukaryotischen Zellen exprimieren. Hierbei können jedoch z.T. auch nicht funktionelle Assoziationen der vier variablen Domänen erfolgen.

Die rekombinante Antikörpertechnologie hat in den letzten Jahren zur Entwicklung neuer kleiner, bivalenter bzw. bispezifischer Antikörperfragmente geführt. Solche Moleküle stellen z.B. die "Diabodies" dar (Holliger et al., Proc. Natl. Acad. Sci. USA 90, 6444-6448, 1993). Dabei handelt es sich um variable VH- und VL-Domänen der Immunglobuline, die durch einen sehr kurzen Linker miteinander verbunden sind. Dieser Linker ist zu kurz, um eine Zusammenlagerung der VH- und VL-Domäne einer Kette zu bewirken, wie es bei einzelkettigen Fv-Fragmenten geschieht. Dadurch lagern sich die VH- und VL-Domänen zweier Ketten zu einem Dimer zusammen, so daß Moleküle mit zwei Bindungsstellen entstehen (Perisic et al., Structure 2, 1217-1226, 1994). Bispezifische "Diabodies" entstehen durch die Expression von zwei Ketten der Struktur VH(A)-VL(B) und VH(B)-VL(A) in einer Zelle. Hierbei bedeutet VL die variable (V) Domäne der leichten (L) Kette und VH die variable Domäne (V) der schweren (H) Kette eines Immunglobulins, wobei diese variablen Domänen das Antigen (A) bzw. (B) binden. Durch die Zusammenlagerung der VH-Teile mit den VL-Teilen entstehen heterodimere Fragmente mit funktionell aktiven Bindungsstellen. Bakteriell exprimierte bispezifische "Diabodies" wurden bereits erfolgreich für die Rekrutierung von verschiedenen Effektormolekülen, Immunglobuline, Clq oder Enzyme bzw. Effektorzellen, wie z.B. zytotoxische T-Lymphozyten, eingesetzt (Kontermann et al., Nature Biotechnol. 15, 629 (1997); Holliger et al., Protein Engin. 9, 299 (1996); Nature Biotechnol. 15, 632 (1997), Zhu et al., BioTechnol. 14, 192 (1996); FitzGerald et al., Protein Engin. 10, 1221 (1997); Krebs et al., J. Biol. Chem. 273, 2858 (1998)).

"Diabodies" haben jedoch folgende Nachteile:
Da die beiden VH(A)-VL(B) und VH(B)-VL(A) Ketten physisch nicht miteinander verbunden sind, können in gleichem Maße Homo- wie Heterodimere entstehen, die ein sehr aufwendiges Reinigungsverfahren für die Heterodimere notwendig machen. Des weiteren kommt es zu einer Dissoziation der Dimere, wie es bereits für scFv-Fragmente gezeigt wurde (Glockshuber et al., Biochem. 29, 1362-1367 (1990)). Zur Lösung dieser Probleme wurden Disulfid-stabilisierte "Diabodies" (FitzGerald et al., Protein Engin. 10, 1221-1225, 1997) oder "knob-into-hole-Diabodies" (Zhu et al., Protein Sci. 6, 781-788, 1997) entwickelt. Deren Herstellung ist jedoch mit einem erheblichen Aufwand verbunden. Zudem wird für die gentechnische Expression eines bispezifischen "Diabody" für jede Kette eine Signalsequenz und eine Ribosomenbindestelle benötigt, was sehr aufwendig ist. Zusätzlich können nicht äquimolare Mengen der variablen Domänen exprimiert werden, was den Anteil an nicht funktionellen Homodimeren erhöht.

Aufgabe der vorliegenden Erfindung war daher, mehrfach-antigenbindende Moleküle zu finden, die die beschriebenen Nachteile der sogenannten "Diabodies" nicht haben und die in überwiegend homogener Form auf einfach Art und Weise hergestellt werden können.

Ein Gegenstand der vorliegenden Erfindung ist daher ein einzelkettiges, mehrfach-antigenbindendes Molekül enthaltend folgende Komponenten:
a) eine variable Domäne einer schweren Kette eines Immunglobulins (VH) mit einer ersten Spezifität (A) und funktionelle Teile hiervon,
b) eine variable Domäne einer leichten Kette eines Immunglobulins (VL) mit einer zweiten Spezifität (B) und funktionelle Teile hiervon,
c) eine variable Domäne einer schweren Kette eines Immunoglobulins (VH) mit der Spezifität (B) und funktionelle Teile hiervon, sowie
d) eine variable Domäne einer leichten Kette eines Immunglobulions (VL) mit der Spezifität (A) und funktionelle Teile hiervon,
wobei die Domänen VH und VL in Form eines VH-VL-Konstruktes oder VL-VH-Konstruktes und die beiden VH-VL-Konstrukte über ein Peptid (P) verbunden sind.

In einer weiteren Ausführungsform enthält das erfindungsgemäße Molekül mehr als zwei der genannten VH-VL-Konstrukte. Hierdurch können Moleküle erhalten werden, die mehrere Spezifitäten (A), (B), (C) usw. enthalten.

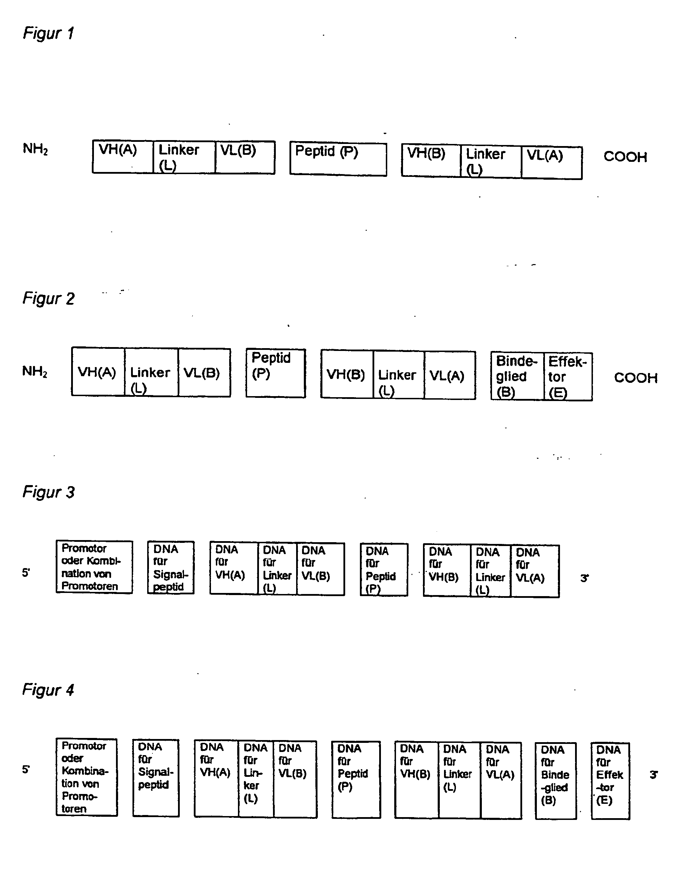

In einer bevorzugten Ausführungsform sind die einzelnen VH-VL-Konstrukte über deren variable Domäne mit gleicher Spezifität gebunden (z.B. VH(B)-peptide-VL(B)). Eine besonders bevorzugte Struktur eines erfindungsgemäßen Moleküls ist in Fig. 1 dargestellt.

In einer weiteren bevorzugten Ausführungsform sind die einzelnen Spezifitäten im wesentlichen identisch. Der Begriff "im wesentlichen identisch" bedeutet gemäß der vorliegenden Erfindung, daß die molekulare Struktur der Domänen durchaus unterschiedlich sein kann, deren Spezifität, d.h. die spezifische antigenbindende Funktion jedoch erhalten bleibt. Hieraus ergibt sich, daß das erfindungsgemäße Molekül nicht nur eine vollständige variable Domäne einer schweren bzw. leichten Kette eines Immunoglobulins enthalten kann, sondern auch deren funktionelle Teile, d.h. die Teile, die ihre spezifische antigenbindende Eigenschaft erhalten haben. Beispielsweise gehören im Sinne der vorliegenden Erfindung hierzu auch künstliche Konstrukte, die aus den einzelnen CDR-Teilen ("complementary determining regions") von variablen Domänen eines Immunglobulins aufgebaut sind (Jones, P.T. et al. Nature, 321, 522, 1986; Riechmann, L. et al. Nature 332, 323, 1988; Verhoeyen, M. et al. Science, 239, 1534, 1988).

In einer bevorzugten Ausführungsform sind die Domänen VH und VL über einen Peptid-Linker (L) in Form eines VH-L-VL-Konstruktes oder VL-L-VH-Konstruktes verbunden. Der Linker sollte hierbei im allgemeinen möglichst kurz, vorzugsweise ca. 1 - 20 Aminosäuren insbesondere ca. 1 - 5 Aminosäuren lang sein. Besonders bevorzugt ist ein Linker mit der Sequenz GGGGS (SEQ ID NO: 7).

Im Gegensatz zum Linker (L) kann das verbindende Peptid (P) beliebig lang sein. Bevorzugt ist jedoch ein Peptid (P) mit ca. 12 - 40 Aminosäuren, insbesondere mit ca. 12 - 20 Aminosäuren, vor allem mit ca. 14 Aminosäuren. Besonders bevorzugt ist ein Peptid (P) das die Aminosäuresequenz GGGGSGGRASGGGS (SEQ ID NO: 1) oder GGGGSGGRASGGGGS (SEQ ID NO: 2) enthält und insbesondere ein Peptid (P), das aus der genannten Aminosäuresequenz besteht.

In einer weiteren bevorzugten Ausführungsform enthält das erfindungsgemäße Molekül als eine weitere Komponente einen Effektor (E). Dieser Effektor kann an das erfindungsgemäße Molekül direkt oder gegebenenfalls über ein Bindeglied (B) gebunden sein. Besonders bevorzugt ist es, wenn das Bindeglied (B) eine Protease-Spaltsequenz, vorzugsweise eine PSA (Prostataspezifisches Antigen)-, Cathepsin-, Plasminogen- und/oder Plasminogenaktivator-Spaltsequenz enthält.

Die Protease-Spaltsequenz ist deshalb besonders bevorzugt, da hierdurch der Effektor von dem erfindungsgemäßen Molekül mit Hilfe einer Protease abgetrennt werden kann. Die Trennung ist besonders dann vorteilhaft, wenn durch direkte oder über das Bindeglied vermittelte indirekte Bindung des Effektors an das erfindungsgemäße Molekül die Aktivität des Effektors inhibiert wird.

Da Proteasen besonders in Bereichen von Entzündungen oder in Tumoren vorhanden sind, werden dort die Effektoren, welche vorzugsweise aufgrund ihrer Bindung über eine Protease-Spaltsequenz an das erfindungsgemäße Molekül inaktiviert sind, in hohem Maße lokal freigesetzt. Durch die Wahl geeigneter Zielstrukturen für das erfindungsgemäße Molekül, beispielsweise mit einer Spezifität für Antigene auf Tumorzellen, für Antigene auf Tumorassoziierten Endothelzellen oder für Antigene auf Entzündungszellen, wie z.B. Lymphozyten oder Makrophagen, kann zusätzlich eine Anreicherung des erfindungsgemäßen Moleküls mit dem Effektor im Bereich beispielsweise einer Entzündung oder eines Tumors erreicht werden.

Gemäß der vorliegenden Erfindung können auch mehrere Effektoren an dem erfmdungsgemäßen Molekül direkt oder indirekt gebunden sein.

Proteasen oder die dazugehörigen Spaltsequenzen sind beispielsweise in DE 19704301.1 im Detail aufgeführt.

Ein Beispiel eines bevorzugten erfindungsgemäßen Moleküls mit einem Effektor, der über ein Bindeglied an das Molekül gebunden ist, ist in Fig. 2 schematisch dargestellt.

Die Auswahl der einzelnen Komponenten des erfindungsgemäßen Moleküls richtet sich im allgemeinen nach dem Anwendungsgebiet.

Soll das erfindungsgemäße Molekül als Diagnostikum verwendet werden, so ist beispielsweise in einer weiteren Ausführungsform die erste Spezifität (A) gegen ein zu analysierendes Molekül gerichtet und die zweite Spezifität (B) direkt oder indirekt gegen einen Analyten. Beispielsweise kann der Analyt ein radioaktives Molekül, ein fluoreszierendes Molekül oder ein Enzym, welches durch seine enzymatische Aktivität eine Vorstufe eines Analyten in einen aktiven Analyten überführt, sein.

In einer weiteren bevorzugten Ausführungsform ist die erste Spezifität (A) gegen ein zu analysierendes Molekül gerichtet, die zweite Spezifität (B) gegen ein anderes zu analysierendes Molekül und der Effektor (E) ist ein Analyt, beispielsweise ein radioaktives Molekül, ein fluoreszierendes Molekül und/oder ein Enzym, wie oben bereits näher erläutert.

Das erfindungsgemäße Molekül kann jedoch auch als Ligand für die zielzellspezifische Bindung eines viralen oder nichtviralen Vektors verwendet werden. In einer besonderen Ausführungsform kann das erfindungsgemäße Molekül als sogenannter multifunktioneller Ligand verwendet werden. Hierzu ist es vorteilhaft, wenn das Peptid (P) und/oder der Effektor (E) ein fusiogenes Peptid enthält. Der multifunktionelle Ligand dient zur zielzellspezifischen Übertragung von Nukleotidsequenzen und ist im allgemeinen ein Protein, welches einen zielzellspezifischen Liganden, einen Genkonstrukt-spezifischen Liganden und ein fusiogenes Peptid enthält. Mit Hilfe derartiger multifunktioneller Liganden können Genkonstrukte, d.h. Nukleinsäurekonstrukte, spezifisch an eine Zielzelle gebunden werden und das fusiogene Peptid ermöglicht die Penetration des Nukleinsäurekonstruktes durch die Zellmembran hindurch in den Zellkern und damit auch die Freisetzung aus dem Endosom.

Bei der Verwendung des erfindungsgemäßen Moleküls als Liganden für einen Vektor oder als multifunktionellen Liganden ist es vorteilhaft, wenn die erste Spezifität (A) gegen eine Zielzelle gerichtet ist und die zweite Spezifität (B) gegen einen Vektor. Der Vektor ist im allgemeinen eine Nukleinsäure, ein kationisches Peptid oder Protein, ein kationisches Lipid, ein kationisches Polymer oder ein kationisches Porphyrin. In einer besonderen Ausführungsform dieser Erfindung ist der Vektor ein viraler Vektor, abgeleitet beispielsweise von Adenoviren (AdV), Adeno-assoziierten Viren (AAV), Vaccinia Virus, RSV, HSV, Influenza-Virus, oder Lentivirus.

Beispiele für zielzellspezifische Liganden, für Membranstrukturen auf der Zielzelle, für zielzellspezifische Liganden und für Genkonstrukt-spezifische Liganden, welche sich von Immunglobulinen ableiten, d.h. VH- und VL-Domänen enthalten, wie auch von Peptiden mit fusiogener Eigenschaft sind im Detail in der DE 19649645.4 beschrieben.

Das erfindungsgemäße Molekül eignet sich auch zur Prophylaxe und/oder als Therapeutikum.

Hierzu ist beispielsweise die erste Spezifität (A) gegen eine Zellmembran, wie beispielsweise gegen Lymphozyten, Makrophagen, Monozyten, Granulozyten, hämatopoietische Zellen, Endothelzellen, glatte Muskelzellen, quergestreifte Muskelzellen, Epithelzellen, Leberzellen, Nierenzellen, Gliazellen, Zellen des Stützgewebes, Tumorzellen oder Leukämiezellen, oder gegen Proteine der extrazellulären Matrix, des Komplementsystems, des Gerinnungssystems, des Kininsystems, des Blutplasmas, des Stützgewebes, oder gegen Cytokine oder Chemokine, oder gegen körpereigene oder körperfremde Toxine, oder gegen Arzneimittel, beispielsweise Digitalis, und/oder gegen Infektionserreger, wie z.B. bakterielle, virale und/ oder parasitäre Infektionserreger, gerichtet.

Die zweite Spezifität (B) kann beispielsweise gegen eine Zellmembran von z.B. Lymphozyten, Makrophagen, Monozyten oder Granulozyten gerichtet sein, mit der Folge, daß deren Vernetzung mit einer Zielstruktur dort zu zytotoxischen, immunmodulierenden oder entzündlichen Vorgängen führen kann.

Sie kann auch gegen Cytokine, Chemokine oder Wachsturnsfaktoren gerichtet sein mit der Folge, daß deren Vernetzung mit einer Zielstruktur dort immunmodulierende oder proliferative Vorgänge auslösen kann. Sie kann auch gegen Proteine des Komplementsystems gerichtet sein, welche dessen Aktivierung initiieren, verstärken oder inhibieren. Die Vernetzung eines solchen Proteins mit einer Zielstruktur kann dort je nach Wahl des Proteins entzündliche und zytolytische bzw. antientzündliche und zytoprotektive Reaktionen auslösen. Sie kann auch gegen Proteine des Gerinnungssystems gerichtet sein, welche dessen Aktivierung initiieren, verstärken oder inhibieren. Die Vernetzung eines solchen Proteins mit der Zielstruktur kann je nach Wahl des Proteins Thrombosen induzieren oder verhindern. Weiter kann sie gegen fibrinolytische Proteine, welche zur Auflösung von Fibringerinnsel an der Zielstruktur führen, gegen Enzyme, welche an der Zielstruktur die unwirksame Vorstufe eines Wirkstoffes in einen aktiven, z. B. zytotoxischen Wirkstoff überführen können, gegen Peptidhormone oder Steroidhormone, gegen den konstanten Teil eines Immunglobulins, gegen einen Mediator, wie beispielsweise Histamin, Serotonin, Leukotrien, Prostacyclin oder Kinin, gegen Infektionserreger, wie z.B. bakterielle, virale und/oder parasitäre Infektionserreger oder gegen Tumorzellen gerichtet sein.

Cytokine als Zielstrukturen oder Monozyten, Makrophagen und/oder Lymphozyten als Zielstrukturen können durch das erfindungsgemäße Molekül mit Infektions- oder Tumorantigenen vernetzt werden und hierdurch in vivo eine verstärkte Immunreaktion gegen das Antigen auslösen. Vorteilhaft ist es hierbei, zu dem erfindungsgemäßen Molekül das jeweilige Antigen vom Infektionserreger oder vom Tumor und gegebenenfalls auch das Cytokin hinzuzugeben und den vernetzten Komplex lokal zu verabreichen oder zu injizieren.

Die zweite Spezifität (B) kann auch gegen körpereigene oder körperfremde Toxine gerichtet sein, so daß entweder eine Neutralisierung des Toxins und Phagozytose oder aber eine toxische Reaktion auf die Zielstruktur bewirkt werden kann. Weiter kann sie gegen Arzneimittel, wie z.B. Digitalis, gerichtet sein, so daß eine Komplexierung und Elimination des Arzneimittels bewirkt werden kann.

In einer weiteren bevorzugten Ausführungsform erlaubt das erfindungsgemäße Molekül mit Effektor die Vernetzung zweier gleicher oder unterschiedlicher Zielstrukturen mit einem oder mehreren Effektor/en.

Als Effektoren eignen sich beispielsweise eine Transmembrandomäne, ein Glykophospholipidanker, der den Liganden bindende Teil eines Rezeptors; der Ligand für einen Rezeptor oder die den Rezeptor bindende Teilsequenz des Liganden; ein Peptidhormon; ein Cytokin; ein Wachstumsfaktor; ein Wachstumsfaktorinhibitor; ein Chemokin; ein Interferon; ein Mediator; ein kreislaufwirksames Peptid; ein Enzym, welches eine inaktive Vorstufe eines Wirkstoffes in einen aktiven Wirkstoff überführt; ein Protein, welches die Gerinnung aktiviert oder inhibiert; ein Protein, welches die Fibrinolyse aktiviert oder inhibiert; ein Protein, welches das Komplementsystem aktiviert oder inhibiert; eine oder mehrere konstante Domänen eines Immunglobulins; ein zytotoxisches Peptid; ein anderes, einzelkettiges, einfach- oder mehrfach-, insbesondere zweifach-antigenbindendes Molekül; ein Tumorantigen oder das Antigen eines Infektionserregers, wie beispielsweise ein bakterielles Antigen oder ein virales Antigen; ein Peptid enthaltend Cystein zur Herstellung von Dimeren des erfindungsgemäßen Moleküls; und/oder ein di- bzw. multimerisierendes Peptid (Plückthun und Pack, Immunotechnol. 3, 83-105 (1997)).

Ein anderer Gegenstand der vorliegenden Erfindung ist eine Nukleinsäure kodierend für ein erfindungsgemäßes Molekül. Die Nukleinsäure ist im allgemeinen eine DNA oder RNA, vorzugsweise eine doppelsträngige DNA.

Für die gegebenenfalls gewünschte Sekretion des erfindungsgemäßen Expressionsproduktes enthält die erfindungsgemäße Nukleinsäure am 5'-Ende eine Nukleotidsequenz kodierend für eine Signal- oder Transmembransequenz (siehe z.B. DE19639103.2 oder DE19651443.6). Ein Beispiel für geeignete Signal- oder Transmembransequenzen ist die Signalsequenz für das Immunglobulin (DNA-Position s 63 bis ≥ 107), die Signalsequenz für das CEA (DNA-Position ≤ 33 bis ≥ 134) oder die Signalsequenz des Human Respiratory Syncytial Virus Glycoproteins (cDNA der Aminosäuresequenzen ≤ 38 bis ≥ 50 oder 48 bis 65). -

In einer weiteren Ausführungsform enthält die erfindungsgemäße Nukleinsäure am 5'-Ende einen Promotor und/oder Aktivator. Der Aktivator ist vorzugsweise zellspezifisch, zellzyklusspezifisch, metabolisch-spezifisch und/oder durch einen Wirkstoff aktivierbar oder supprimierbar. Derartige Aktivatorsequenzen einschließlich ihrer Kombinationen sind in EP 0 790 313, DE 196 17 851.7, DE 196 39 103.2, DE 196 51 443.6, DE 197 04 301.1, EP 0 860 445 bzw. DE 197 10 643.9 beschrieben. Besonders bevorzugte erfindungsgemäße Nukleinsäurekonstrukte sind in den Fig. 3 und 4 schematisch dargestellt.

In einer bevorzugten Ausführungsform enthält die erfindungsgemäße Nukleinsäure am 5'-Ende des Startkodons die Sequenz GCCACC oder GCCGCC, welche eine Verstärkung der Translation bewirken kann.

Eine andere Ausführungsform der vorliegenden Erfindung bezieht sich auf einen Vektor enthaltend die erfindungsgemäße Nukleinsäure. Der Vektor kann hierbei ein viraler oder nicht-viraler Vektor sein, vorzugsweise ein nicht-viraler Vektor, der insbesondere ausgewählt ist aus einem kationischen Lipid, einem kationischen Polymer, einem kationischen Peptid oder einem kationischen Porphyrin.

Zur Herstellung der erfindungsgemäßen Moleküle wird die beschriebene Nukleinsäure in einen Expressionsvektor, beispielsweise ein geeignetes Plasmid kloniert, in eine geeignete Zelle, beispielsweise in eine Bakterien-, Hefe-, Insekten- oder Säugerzelle eingebracht, die so transformierte oder transfizierte Zelle kultiviert und das Expressionsprodukt gegebenenfalls isoliert. Die Methoden sind dem Fachmann allgemein bekannt und beispielsweise bei Sambrook J. et al. Molecular Cloning, A Laboratory Handbook 2nd ed., Cold Spring Harbor Laboratory Press, 1989 näher beschrieben.

In einer besonderen Ausführungsform der vorliegenden Erfindung exprimieren diese Zellen ein erfindungsgemäßes Molekül mit einem Effektor, wobei dieser Effektor vorzugsweise eine Transmembrandomäne ist.

So kann beispielsweise die DNA-Sequenz kodierend für die Transmembransequenz des menschlichen Makrophagenkolonie-stimulierenden Faktors (DNA-Position ≤ 1485 bis ≥ 1554) oder die DNA-Sequenz kodierend für die Signal- und Transmembranregion des menschlichen Respiratory Syncytial Virus (RSV)-Glykoproteins G (Aminosäuren 1 bis 63 oder deren Teilsequenz Aminosäuren 38 bis 63) oder die DNA-Sequenz kodierend für die Signal- und Transmembranregion der Influenzavirus-Neuraminidase (Aminosäuren 7 bis 35 oder die Teilsequenz Aminosäuren 7 bis 27) zwischen der Promotorsequenz und der DNA-Sequenz des erfindungsgemäßen Moleküls oder auch am 3' Ende des Gens eingefügt werden.

Zur Verankerung des Wirkstoffes in die Zellmembran der das erfindungsgemäße Molekül exprimierenden Zellen kann jedoch auch eine Nukleotidsequenz kodierend für einen Glykophospholipid-Anker in das Nukleinsäurekonstrukt eingefügt werden.

Die Einfügung eines Glykophospholipid-Ankers erfolgt im allgemeinen am 3' Ende der Nukleotidsequenz kodierend für das erfindungsgemäße Molekül und kann zusätzlich zur Einfügung einer Signalsequenz erfolgen.

Glykophospholipid-Anker sind beispielsweise für das CEA, für das N-CAM und für weitere Membranproteine, wie beispielsweise Thy-1, beschrieben worden.

Durch diese Transmembranregion exprimiert die jeweilige Zelle das erfindungsgemäße Molekül auf der Zellmembran und erhält somit einen "Rezeptor", der für diejenigen Ziel- oder Effektorstrukturen spezifisch ist, welche durch die antigenbindenden Teile des erfindungsgemäßen Moleküls erkannt werden.

Ein anderer bevorzugter Rezeptor ist die transmembran- bzw. signaltransduzierende Domäne eines Rezeptors, beispielsweise der T-Zellrezeptor oder der M-CSF-Rezeptor. Hierdurch kann die das erfindungsgemäße Molekül auf der Zellmembran exprimierende Zelle durch Bindung der Zielstrukturen zu spezifischen Funktionen aktiviert werden. Derartige spezifische Funktionen können beispielsweise zytotoxische Reaktionen von T-Lymphozyten, Phagozytosereaktionen von Makrophagen und Granulozyten oder Exozytosereaktionen von Granulozyten, Monozyten und Makrophagen sein.

Ein anderer Gegenstand der vorliegenden Erfindung ist daher eine Zelle enthaltend eine erfindungsgemäße Nukleinsäure oder einen erfindungsgemäßen Vektor, insbesondere eine Bakterien-, Insekten-, Hefe- oder Säugerzelle. Als Säugerzellen sind neben den allgemein bekannten Zellen zur Expression von Nukleinsäuren, wie z.B. CHO- oder BHK-Zellen, auch ein Lymphozyt, ein Makrophage, eine Gliazelle, eine Epithelzelle, eine Leberzelle, eine Nierenzelle, eine Knochenmarkszelle, eine Endothelzelle, eine glatte oder quergestreifte Muskelzelle oder ein Fibroblast geeignet.

Die zuletzt genannten Zellen sind insbesondere für eine gentherapeutische Anwendung geeignet, da diese Zellen, welche das erfindungsgemäße Nukleinsäurekonstrukt enthalten, einem Patienten lokal oder parenteral, z.B. intravenös, intraarteriell, in eine Körperhöhle, in ein Organ oder subkutan injiziert werden können, um so zur Prophylaxe oder Therapie einer Erkrankung zu dienen.

Für die gentherapeutische Behandlung von Erkrankungen können jedoch auch die erfindungsgemäßen Nukleinsäurekonstrukte direkt dem Patienten lokal, in eine Körperhöhle, in ein Organ, in das Blutgefässsystem, subkutan oder intramuskulär verabreicht werden.

Ein anderer Gegenstand der vorliegenden Erfindung ist daher auch ein Arzneimittel enthaltend ein erfindungsgemäßes Molekül, eine erfindungsgemäße Nukleinsäure einen erfindungsgemäßen Vektor oder eine erfindungsgemäße Zelle sowie ein Diagnostikum enthaltend ein erfindungsgemäßes Molekül, welches auch gegen einen Analyten gerichtet ist, wie oben bereits näher beschrieben.

Das erfindungsgemäße Molekül, die erfindungsgemäße Nukleinsäure, der erfindungsgemäße Vektor oder die erfindungsgemäße Zelle eignen sich somit zur Therapie, Prophylaxe oder Diagnose von beispielsweise Tumorerkrankungen, Autoimmunerkrankungen, Entzündungserkrankungen, Erkrankungen des Blutes, insbesonderes des Blutgerinnungs- und/oder Blutkreislaufsystems, Erkrankungen des Nervensystems und/oder Infektionserkrankungen.

Die Wahl der einzelnen Komponenten richtet sich hierbei im allgemeinen nach der Verwendung der erfindungsgemäßen Gegenstände. Im folgenden werden die einzelnen Komponenten und deren Verwendungen allgemein und beispielhaft näher beschrieben:

Für die Herstellung eines erfindungsgemäßen Gegenstandes kann, wie bereits in Figur 3 und 4 beispielhaft dargestellt, das erfindungsgemäße Nukleinsäurekonstrukt an seinem 5'-Ende mit dem 3'-Ende einer Nukleinsäure kodierend für eine Signalsequenz und diese wiederum an ihrem 5'-Ende mit dem 3'-Ende einer Promotorsequenz verknüpft.

Zu den auszuwählenden Promotorsequenzen gehören beispielsweise uneingeschränkt aktivierbare Promotoren und Aktivatorsequenzen, wie beispielsweise der Promotor der RNA-Polymerase III, der Promotor der RNA-Polymerase II, der CMV-Promotor und/oder -Enhancer, der SV40 Promotor; oder virale Promotor- und Aktivatorsequenzen, wie beispielsweise HBV, HCV, HSV, HPV, EBV, HTLV, HIV.

Bei Verwendung des HIV-Promotors wird vorzugsweise die gesamte LTR-Sequenz einschließlich der TAR-Sequenz [Position ≤ -453 bis ≥ -80, Rosen et al., Cell 41, 813 (1985)] als virusspezifischer Promotor eingesetzt.

Andere auszuwählende Promotorsequenzen sind z.B. metabolisch aktivierbare Promotor- und Enhancersequenzen, wie beispielsweise der durch Hypoxie induzierbare Enhancer, zellzyklusspezifisch aktivierbare Promotoren, wie beispielsweise der Promotor des cdc25C Gens, des cdc25B Gens, des Cyclin A Gens, des cdc2 Gens, des B-myb Gens, des DHFR-Gens oder des E2F-1 Gens oder aber Bindesequenzen für zellzyklusspezifisch auftretende oder aktivierte Transkriptionsfaktoren. Zu diesen Bindesequenzen gehören beispielsweise Bindesequenzen für c-myc Proteine. Zu diesen Bindesequenzen sind Monomere oder Multimere der als Myc E-Box bezeichneten Nukleotidsequenz [5'-GGAAGCAGACCACGTGGTCTGCTTCC-3'; SEQ ID NO: 3] zu zählen.

Weitere auszuwählende Promotorsequenzen sind z.B. Tetrazyklin aktivierbare Promotoren, wie beispielsweise der Tetrazyklin-Operator in Kombination mit einem entsprechenden Repressor, oder Chimäre Promotoren. Ein chimärer Promotor stellt die Kombination einer stromaufwärts gelegenen zellspezifisch, metabolisch oder virusspezifisch aktivierbaren Aktivatorsequenz mit einem stromabwärts gelegenen Promotormodul dar, welches die Nukleotidsequenz CDE-CHR oder E2FBS-CHR enthält, an welche suppressive Proteine binden, die hierdurch die Aktivierung der stromaufwärts gelegenen Aktivatorsequenz in G₀- und G₁-Phase des Zellzyklus hemmen können (WO96/06943; Lucibello et al., EMBO J. 14, 132 (1995)).

Andere auszuwählende Promotorsequenzen sind z.B. zellspezifisch aktivierbare Promotoren, wie vorzugsweise Promotoren oder Aktivatorsequenzen aus Promotoren oder Enhancern von solchen Genen, die für Proteine kodieren, die bevorzugt in ausgewählten Zellen gebildet werden.

Zum Beispiel sind im Sinne der Erfindung in folgenden Zellen Promotoren für folgende Proteine bevorzugt zu verwenden:

Promotor- und Aktivatorsequenzen, die in Endothelzellen aktiviert werden, sind genregulatorische Sequenzen aus Genen, die beispielsweise für folgende Proteine kodieren: Hirn-spezifischer, endothelialer Glucose-1-Transporter, Endoglin, VEGF-Rezeptor-1 (flt-1), VEGF-Rezeptor-2 (flk-1, KDR), til-1 oder til-2, B61-Rezeptor (Eck-Rezeptor), B61, Endothelin, im speziellen Endothelin B oder Endothelin-1, Endothelin-Rezeptoren, insbesondere der Endothelin B-Rezeptor, Mannose-6-Phosphat-Rezeptoren, von Willebrand Faktor, IL-1α, IL-1β, IL-1-Rezeptor, Vascular Cell Adhesion Molecule (VCAM-1) oder synthetische Aktivatorsequenzen, die aus oligomerisierten Bindestellen für Transkriptionsfaktoren, die preferentiell oder selektiv in Endothelzellen aktiv sind, bestehen. Ein Beispiel hierfür ist der Transkriptionsfaktor GATA-2, dessen Bindestelle im Endothelin-1 Gen 5'-TTATCT-3' ist.

Promotoren oder Aktivatorsequenzen, die in Zellen in Nachbarschaft aktivierter Endothelzellen aktiviert werden, sind genregulatorische Sequenzen aus Genen, die beispielsweise für folgende Proteine kodieren: VEGF, wobei die genregulatorischen Sequenzen für das VEGF-Gen die 5' flankierende Region, die 3' flankierende Region, das c-Src-Gen oder das v-Src-Gen sind, oder Steroid-Hormonrezeptoren und deren Promotorelemente, insbesondere der Maus-Mammatumor-Virus-Promotor.

Promotoren oder Aktivatorsequenzen, die in Muskelzellen, insbesondere glatten Muskelzellen aktiviert werden, sind genregulatorische Sequenzen aus Genen, die beispielsweise für folgende Proteine kodieren: Tropomyosin, α-Actin, α-Myosin, Rezeptor für PDGF, Rezeptor für FGF, MRF-4, Phosphofructokinase A, Phosphoglyceratemutase, Troponin C, Myogenen, Rezeptoren für Endothelin A, Desmin, VEGF (s.o.), "artifizielle" Promotoren, oder Promotoren muskelspezifischer Transkriptionsfaktoren, wie Faktoren der Helix-Loop-Helix (HLH)-Familie (MyoD, Myf-5, Myogenen, MRF4) oder das Zinkfingerprotein GATA-4.

Die HLH-Proteine sowie GATA-4 zeigen muskelspezifische Transkription nicht nur mit Promotoren von muskelspezifischen Genen, sondern auch im heterologen Kontext, so auch mit artifiziellen Promotoren. Derartige artifizielle Promotoren sind beispielsweise multiple Kopien der (DNA) Bindestelle für muskelspezifische HLH-Proteine wie der E-Box (Myo D) (z.B. 4x AGCAGGTGTTGGGAGGC; SEQ ID NO: 4) oder multiple Kopien der DNA Bindestelle für GATA-4 des a-Myosin-Heavy Chain Gens (z.B. 5'-GGCCGATGGGCAGATAGAGGGGGCCGATGGGCAGATAGAGG3'; SEQ ID NO: 5)

Promotoren und Aktivatorsequenzen, die in Gliazellen aktiviert werden, sind z.B. genregulatorische Sequenzen, aus Genen, die beispielsweise für folgende Proteine kodieren: das Schwannzell-spezifische Protein Periaxin, Glutaminsynthetase, das Gliazell-spezifische Protein (Glial fibrillary acid protein = GFAP), das Gliazellprotein S100b, IL-6, CNTF, 5-HT-Rezeptoren, TNFα, IL-10, Insulin-like Growth Factor Receptor I and II oder VEGF (s.o.)

Promotoren und Aktivatorsequenzen, die in blutbildenden Zellen aktiviert werden, sind z.B. Promotorsequenzen für Gene eines Cytokins oder seines Rezeptors, die in blutbildenden Zellen oder in benachbarten Zellen, wie beispielsweise dem Stroma, exprimiert sind.

Hierzu gehören Promotorsequenzen aus Genen, die beispielsweise für folgende Cytokine und ihre Rezeptoren kodieren: Stem Cell Factor-Receptor, Stem Cell Factor, IL-1α, IL-1-Rezeptor, IL-3, IL-3-Rezeptor (α-subunit), IL-3-Rezeptor (β-subunit), IL-6, IL-6-Rezeptor, GM-CSF, GM-CSF-Rezeptor (α-Kette), Interferon Regulatory Factor 1 (IRF-1), wobei der Promotor von IRF-1 durch IL-6 gleichermaßen aktiviert wird wie durch IFNγ oder IFNβ, Erythropoietin oder Erythropoietin-Rezeptor.

Promotoren und Aktivatorsequenzen, die in Lymphozyten und/oder Makrophagen aktiviert werden, sind beispielsweise die Promotor- und Aktivatorsequenzen der Gene kodierend für Cytokine, Cytokinrezeptoren und Adhäsionsmoleküle und Rezeptoren für das Fc-Fragment von Antikörpern, wie z.B. IL-1-Rezeptor, IL-1α, IL-1β, IL-2, IL-2-Rezeptor, IL-3, IL-3-Rezeptor (α-subunit), IL-3-Rezeptor (β-subunit), IL-4, IL-4-Rezeptor, IL-5, IL-6, IL-6-Rezeptor, Interferon Regulatory Factor 1 (IRF-1), wobei der Promotor von IRF-1 durch IL-6 gleichermaßen aktiviert wird wie durch IFNγ oder IFNβ, IFNγ Responsive Promotor, IL-7, IL-8, IL-10, IL-11, IFNγ, GM-CSF, GM-CSF-Rezeptor (α-Kette), IL-13, LIF, Makrophagen-Colony Stimulating Factor (M-CSF)-Rezeptor, Typ I und II Makrophagen Scavenger Rezeptoren, MAC-1 (Leukozytenfunktionsantigen), LFA-1α (Leukozytenfunktionsantigen) oder p150,95 (Leukozytenfunktionsantigen).

Promotor- und Aktivatorsequenzen, die in Synovialzellen aktiviert werden, sind beispielsweise die Promotorsequenzen von Genen kodierend für Matrix-Metalloproteinasen (MMP), wie z.B. MMP-1 (interstitielle Kollagenase), MMP-3 (Stromelysin/Transin) oder Tissue Inhibitors of Metalloproteinases (TIMP), wie TIMP-1, TIMP-2, TIMP-3.

Promotoren und Aktivatorsequenzen, die in Leinkämiezellen aktiviert werden, sind beispielsweise Promotoren von Genen, die für folgende Proteine kodieren: HSP-70, bcl-1/cyclin D-1, bcl-2, IL-6, IL-10, TNFα, TNFβ, HOX-11, BCR-Ab1, E2A-PBX-1, PML-RAR (Promyelocytic Leukemia - Retinoic Acid Receptor) oder c-myc, wobei c-myc-Proteine an und aktivieren Multimere der als Myc E-Box bezeichneten Nukleotidsequenz (5'-GGAAGCAGACCAGCTGGTCTGCTTCC-3'; SEQ ID NO: 6) binden.

Promotoren oder Aktivatorsequenzen, die in Tumorzellen aktiviert werden, sind z.B. genregulatorische Nukleotidsequenzen, mit der Transkriptionsfaktoren, gebildet oder aktiv in Tumorzellen, interagieren.

Im Sinne dieser Erfindung zählen zu den bevorzugten Promotoren oder Aktivatorsequenzen genregulatorische Sequenzen bzw. Elemente aus Genen, die besonders in Krebszellen oder Sarkomzellen gebildete Proteine kodieren. So wird bei kleinzelligen Bronchialkarzinomen bevorzugt der Promotor des N-CAM-Proteins, bei Ovarialkarzinomen der Promotor des "Hepatitis growth factor"-Rezeptors oder des L-Plastin und bei Pankreaskarzinomen der Promotor des L-Plastins oder des polymorphen epithelialen Mucins (PEM) verwendet.

Ein wesentlicher Vorteil der vorliegenden Erfindung ist, daß durch die Verknüpfung der einzelnen Komponenten eine heterodimere Assoziation begünstigt wird, so daß überwiegend einzelkettige, mehrfach-antigenbindende Moleküle entstehen. Ferner wird die Dissoziation der Dimere, wie es bereits für scFv-Fragmente gezeigt werden konnte (s. z.B. Glockshuber et al., Biochem. 29, 1362 - 1367, 1990) reduziert. Somit können die erfindungsgemäßen Moleküle weniger aufwendig und in homogenerer Form hergestellt werden als die sogenannten "Diabodies", selbst wenn diese disulfidstabilisiert sind oder in Form von "knob-into-hole-Diabodies" vorliegen.

Desweiteren ist für die Herstellung des erfindungsgemäßen Moleküls nur eine Signalsequenz und eine Ribosomenbindestelle (RBS) notwendig. Im Gegensatz hierzu werden bei den erfindungsgemäßen "Diabodies" für jede Kette eine Signalsequenz und eine RBS benötigt. Ein weiterer Vorteil der vorliegenden Erfindung ist, daß bei den erfindungsgemäßen Molekülen äquimolare Mengen der variablen Domänen exprimiert werden, während bei der Expression der zwei Ketten von "Diabodies" nicht äquimolare Mengen entstehen können und dadurch der Anteil an nichtfunktionellen Homodimeren erhöht wird.

Ein weiterer Vorteil des erfindungsgemäßen Moleküls ist, daß es sich einfach und in funktioneller Form sowohl in Bakterien wie auch in Hefen, Baculoviren sowie eukaryotischen Zellen exprimieren lassen. Zudem besteht neben der Sekretion der erfindungsgemäßen Moleküle die Möglichkeit, diese auch intrazellulär bzw. membranständig zu exprimieren (s. z.B. Biocca & Cattaneo, Trends Cell Biol. 5, 248-252 (1995)).

Ein weiterer Vorteil der erfindungsgemäßen Moleküle ist, daß sie sowohl als Diagnostikum, als auch als Wirkstoff zur Prophylaxe und/oder Therapie einer Erkrankung, wie bispezifische Antikörper, breit einsetzbar sind.

Die Gene von Effektoren und Promotorsequenzen sind im Hinblick auf die gewünschte Anwendung und unter Berücksichtigung der zu transduzierenden Zielzelle auszuwählen. Beispielsweise sind bei folgenden Erkrankungen folgende Kombinationen von Promotorsequenzen und Gene für Effektoren zu wählen:

### Therapie von Tumoren

Für die Therapie von Tumoren dienen als Zielzellen beispielsweise proliferierende Endothelzellen, oder der Endothelzelle benachbarte Stromazellen und Muskelzellen, oder Tumorzellen oder Leukämiezellen, als Promotoren beispielsweise endothelzellspezifische und zellzyklusspezifische, oder zellunspezifische oder muskelzellspezifische und zellzyklusspezifische, oder tumorzellspezifische (solide Tumoren, Leukämien) und zellzyklusspezifische Promotoren, und als Effektoren beispielsweise folgende Gene:

Gene von Inhibitoren der Zellproliferation sind zum Beispiel vom Retinoblastomprotein (pRb=p110) oder die verwandten p 107 und p 130 Proteine. Das Retinoblastomprotein (pRb/p110) und die verwandten p107 und p130 Proteine werden durch Phosphorylierung inaktiviert. Bevorzugt sind solche Gene von Zellzyklusinhibitoren zu verwenden, welche Mutationen für die Inaktivierungsstellen der exprimierten Proteine aufweisen, ohne daß diese hierdurch in ihrer Funktion beeinträchtigt werden. Beispiele für diese Mutationen wurden beschrieben für das p 110. In analoger Weise wird die DNA-Sequenz für das p107 Protein oder das p130 Protein mutiert. Desweiteren ist das Gen vom p53 Protein geeignet. Das Protein p53 wird in der Zelle entweder durch Bindung an spezielle Proteine, wie beispielsweise MDM2, oder durch Oligomerisierung des p53 über das dephosphorylierte C-terminale Serin inaktiviert. Bevorzugt wird somit eine DNA-Sequenz für ein p53 Protein verwendet, welches C-terminal um das Serin 392 verkürzt ist. Weitere geeignete Gene sind das Gen vom p21 (WAF-1), vom p16 Protein, von anderen cdk-Inhibitoren, vom GADD45 Protein oder vom bak Protein.

Gene von Gerinnung induzierenden Faktoren und Angiogeneseinhibitoren sind zum Beispiel vom Plasminogenaktivatorinhibitor-1 (PAI-1), PAI-2, PAI-3, Angiostatin, Interferone (IFNα, IFNβ oder IFNγ), Platelet factor 4, IL-12, TIMP-1, TIMP-2, TIMP-3, Leukemia Inhibitory Factor (LIF) oder Tissue Factor (TF) und dessen gerinnungsaktive Fragmente.

Gene von zytostatischen und zytotoxischen Proteinen, sind zum Beispiel vom Perforin, Granzym, IL-2, IL-4, IL-12, Interferone, wie beispielsweise IFN-α, IFNβ oder IFNγ, TNF, wie TNFα oder TNFβ, Oncostatin M, Sphingomyelinase oder Magainin und Magainin-Derivate.

Gene von Induktoren von Entzündungen sind zum Beispiel vom IL-1, IL-2, RANTES (MCP-2) monocyte chemotactic and activating factor (MCAF), IL-8, macrophage inflammatory protein-1 (MIP-1α, -β), neutrophil activating protein-2 (NAP-2), IL-3, IL-5, human leukemia inhibitory factor (LIF), IL-7, IL-11, IL-13, GM-CSF, G-CSF, M-CSF, Cobra venom factor (CVF) oder Teilsequenzen vom CVF, welche dem menschlichen Komplementfaktor C3b funktionell entsprechen, d.h. welche an den Komplementfaktor B binden können und nach Spaltung durch den Faktor D eine C3 Konvertase darstellen, menschlichen Komplementfaktor C3 oder seine Teilsequenz C3b, von Spaltprodukten des menschlichen Komplementfaktors C3, welche funktionell und strukturell dem CVF ähneln, oder von bakteriellen Proteinen, welche Komplement aktivieren oder Entzündungen auslösen, wie beispielsweise Porine von Salmonella typhimurium, "clumping" Faktoren von Staphylococcus aureus, Moduline besonders von gram-negativen Bakterien, "Major outer membrane protein" von Legionellen oder von Haemophilus influenza Typ B oder von Klebsiellen oder M-Moleküle von Streptokokken Gruppe G.

Gene von Enzymen für die Aktivierung von Vorstufen von Zytostatika, sind zum Beispiel von Enzymen, welche inaktive Vorsubstanzen (Prodrugs) in aktive Zytostatika (Drugs) spalten. Derartige Substanzen und die jeweils zugehörigen Prodrugs und Drugs sind bereits von Deonarain et al. (Br. J. Cancer 70, 786 (1994)), Mullen (Pharmac. Ther 63, 199 (1994)) und Harris et al. (Gene Ther 1, 170 (1994)) übersichtlich beschrieben worden. Beispielsweise kann die DNA-Sequenz eines der folgenden Enzyme zu verwenden: Herpes Simplex Virus thymidinkinase, Varizella Zoster Virus Thymidinkinase, bakterielle Nitroreduktase, bakterielle β-Glucuronidase, pflanzliche β-Glucuronidase aus Secale cereale, humane β-Glucuronidase, humane Carboxypeptidase (CB) zum Beispiel CB-A der Mastzelle, CB-B des Pankreas oder bakterielle Carboxypeptidase, bakterielle β-Laktamase, bakterielle Cytosinedeaminase, humane Katalase bzw. Peroxidase, Phosphatase, im besonderen humane alkalische Phosphatase, humane saure Prostataphosphatase oder Typ 5 saure Phosphatase, Oxidase, im besonderen humane Lysyloxidase oder hinmane saure D-Aminooxidaseperoxidase, im besonderen humane Glutathionperoxidase, humane eosinophile Peroxidase oder humane Schilddrüsenperoxidase, β-Galaktosidase oder α-Galaktosidase. Besonders bevorzugt ist eine β-Galaktosidase zur Umwandlung des Prodrug Daunomycin-β-D-galaktopyranosid in das Zytostatikum Daunomycin.

### Therapie von Autoimmunerkrankungen und Entzündungen

Für die Therapie von Autoimmunerkrankungen und Entzündungen dienen als Zielzellen beispielsweise proliferierende Endothelzellen oder Makrophagen und/oder Lymphozyten oder Synovialzellen, als Promotoren beispielsweise endothelzellspezifische und zellzyklusspezifische, oder makrophagen- und/oder lymphozytenspezifische und/oder zellzyklusspezifische, oder synovialzellspezifische und/oder zellzyklusspezifische Promotoren, und als Effektoren beispielsweise folgende Gene:

Gene für die Therapie von Allergien sind zum Beispiel von IFNβ, IFNγ, IL-10, Antikörper bzw. Antikörperfragmenten gegen IL-4, löslichen IL-4-Rezeptoren, IL-12 oder TGFβ.

Gene für die Verhinderung der Abstoßung von transplantierten Organen sind zum Beispiel von IL-10, TGFβ, löslichen IL-1-Rezeptoren, löslichen IL-2-Rezeptoren, IL-1-Rezeptorantagonisten oder lösliche IL-6-Rezeptoren.

Gene für die Therapie von Antikörper-mediierten Autoimmunerkrankungen sind zum Beispiel von TGFβ, IFNα, IFNβ, IFNγ, IL-12, löslichen IL-4-Rezeptoren, löslichen IL-6-Rezeptoren, immunsuppressiven Antikörpern oder deren V_{H} und V_{L}-enthaltende Fragmente.

Gene für die Therapie von zellmediierten Autoimmunerkrankungen sind zum Beispiel von IL-6, IL-9, IL-10, IL-13, TNFα oder TNFβ, IL-13, einem immunsuppressiven Antikörper oder dessen V_{H}- und V_{L}-enthaltende Fragmente.

Gene von Inhibitoren der Zellproliferation, zytostatische oder zytotoxische Proteine und Enzyme für die Aktivierung von Vorstufen von Zytostatika sind bereits oben näher beschrieben worden.

Gene für die Therapie der Arthritis sind beispielweise Strukturgene, deren exprimiertes Protein die Entzündung beispielsweise im Gelenk direkt oder indirekt hemmt und/oder die Rekonstitution von extrazellulärer Matrix (Knorpel, Bindegewebe) im Gelenk fördert.

Hierzu gehören zum Beispiel IL-1-Rezeptorantagonist (IL-1-RA), da IL-1-RA die Bindung von IL-1α,β inhibiert; löslicher IL-1-Rezeptor; da löslicher IL-1-Rezeptor IL-1 bindet und inaktiviert; IL-6, da IL-6 die Sekretion von TIMP und Sinperoxiden erhöht und die Sekretion von IL-1 und TNFα durch Synovialzellen und Chondrozyten vermindert; löslicher TNF-Rezeptor, da löslicher TNF-Rezeptor TNF bindet und inaktiviert; IL-4, da IL-4 die Bildung und Sekretion von IL-1, TNFα und MMP inhibiert; IL-10, da IL-10 die Bildung und Sekretion von IL-1, TNFα und MMP inhibiert und die Sekretion von TIMP erhöht; Insulin-like growth factor (IGF-1), da IGF-1 die Synthese von extrazellulärer Matrix stimuliert; TGFβ, im speziellen TGFβ 1 und TGFβ2, da TGFβ die Synthese von extrazellulärer Matrix stimuliert; Superoxiddismutase oder TIMP, im speziellen TIMP-1, TIMP-2 oder TIMP-3.

### Therapie des blutbildenden Systems

Für die Therapie der mangelhaften Bildung von Zellen des Blutes dienen als Zielzellen beispielsweise proliferierende, unreife Zellen des blutbildenden Systems oder Stromazellen benachbart den blutbildenden Zellen, als Promotoren beispielsweise für blutbildende Zellen spezifische und/oder zellzyklusspezifische, oder zellunspezifische und zellzyklusspezifische Promotoren, und als Effektoren beispielsweise folgende Gene: Gene für die Therapie der Anämie sind zum Beispiel vom Erythropoietin.

Gene für die Therapie der Leukopenie sind zum Beispiel vom G-CSF, GM-CSF oder M-CSF.

Gene für die Therapie der Thrombozytopenie sind zum Beispiel vom IL-3, Leukemia Inhibitory Factor (LIF), IL-11 oder Thrombopoietin.

### Therapie des Nervensystems

Für die Therapie von Schäden des Nervensystems dienen als Zielzellen beispielsweise Gliazellen oder proliferierende Endothelzellen, als Promotoren beispielsweise Gliazell-spezifische und zellzyklusspezifische oder Endothelzellspezifische und zellzyklusspezifische, oder unspezifische und zellzyklusspezifische Promotoren, und als Effektoren beispielsweise folgende Gene:

Gene für neuronale Wachstumsfaktoren sind zum Beispiel vom FGF, Nerve growth factor (NGF), Brain-derived neurotrophic factor (BDNF), Neurotrophin-3 (NT-3), Neurotrophin-4 (NT-4) oder Ciliary neurotrophic factor (CNTF).

Gene für Enzyme sind zum Beispiel Gene der Tyrosinhydroxylase oder Dopadecarboxylase.

Gene für Cytokine und deren Inhibitoren, welche die neurotoxische Wirkung von TNFα inhibieren oder neutralisieren, sind zum Beispiel vom TGFβ; von löslichen TNF-Rezeptoren, da TNF-Rezeptoren TNFα neutralisieren; von IL-10, da IL-10 die Bildung von IFNγ, TNFα, IL-2 und IL-4 inhibiert; von löslichen IL-1-Rezeptoren wie IL-1-Rezeptor I oder IL-1-Rezeptor II, da lösliche IL-1-Rezeptoren die Aktivität von IL-1 neutralisieren; vom IL-1-Rezeptor-Antagonist oder von löslichen IL-6-Rezeptoren.

### Therapie des Blutgerinnungs- und Blutkreislaufsystems

Für die Therapie von Störungen des Blutgerinnungs- und Blutkreislaufsystems dienen als Zielzellen beispielsweise Endothelzellen, proliferierende Endothelzellen, somatische Zellen in Nachbarschaft von Endothelzellen und glatte Muskelzellen oder Makrophagen, als Promotoren beispielsweise zellunspezifische und zellzyklusspezifische, oder für Endothelzellen, glatte Muskelzellen oder Makrophagen spezifische und zellzyklusspezifische Promotoren, und als Effektoren beispielsweise folgende Gene:

Gene für die Inhibition der Gerinnung oder für die Förderung der Fibrinolyse sind zum Beispiel vom Tissue Plasminogen Activator (tPA), Urokinase-type Plasminogen Activator (uPA), von Hybriden von tPA und uPA, vom Protein C, Hirudin, von Serin Proteinase Inhibitoren (Serpine), wie beispielsweise C-1S-Inhibitor, α1-Antitrypsin oder Antithrombin III, oder vom Tissue Factor Pathway Inhibitor (TFPI).

Gene für die Förderung der Gerinnung sind zum Beispiel vom F VIII, F IX, von Willebrand factor, F XIII, PAI-1, PAI-2 oder Tissue Factor and Fragmente hiervon.

Gene für Angiogenesefaktoren sind zum Beispiel vom VEGF oder FGF.

Gene für die Blutdrucksenkung sind zum Beispiel vom Kallikrein oder Endothelzell "nitric oxide synthase".

Gene für die Inhibition der Proliferation von glatten Muskelzellen nach Verletzungen der Endothelschicht sind zum Beispiel von einem antiproliferativen, zytostatischen oder zytotoxischen Protein oder von einem Enzym zur Aufspaltung von Vorstufen von Zytostatika in Zytostatika, wie bereits oben aufgeführt, oder von einem Fusionsprotein eines dieser Wirkstoffe mit einem liganden, beispielsweise einem Antikörper oder Antikörperfragmenten, der für Muskelzellen spezifisch ist.

Gene für weitere Blutplasmaproteine sind zum Beispiel vom Albumin, C1-Inaktivator, Serum Cholinesterase, Transferrin oder 1-Antitrypsin.

### Prophylaxe, und/oder Therapie von Infektionserkrankungen

Für Impfungen dienen als Zielzellen beispielweise Muskelzellen oder Makrophagen und/oder Lymphozyten, als Promotoren beispielsweise unspezifische und zellzyklusspezifische oder zielzellspezifische und zellzyklusspezifische Promotoren, und als Effektoren beispielsweise Gene für die Prophylaxe von Infektionserkrankungen

Als Wirksubstanz wird im allgemeinen die DNA eines vom Infektionserreger gebildeten Proteins ausgewählt, welches durch Auslösen einer Immunreaktion, d.h. durch Antikörperbindung und/oder durch zytotoxische T-Lymphozyten, zur Neutralisierung und/oder zur Abtötung des Erregers führt. Derartige sogenannte Neutralisationsantigene werden als Impfantigene bereits angewandt (siehe z.B. Ellis, Adv. Exp. Med. Biol. 327, 263 (1992).

Bevorzugt im Sinne der Erfindung ist eine Nukleinsäure kodierend für Neutralisationsantigene folgender Erreger: Influenza A-Virus, HIV, Tollwut-Virus, HSV (Herpes Simplex Virus), RSV (Respiratory Syncytial Virus), Parainfluenza-Virus, Rotavirus, VZV (Varizella Zoster Virus), CMV (Cytomegalo-Virus), Masern-Virus, HPV (Humanes Papillomvirus), HBV (Hepatitis B-Virus), HCV (Hepatitis C-Virus), HDV (Hepatitis D-Virus), HEV (Hepatitis E-Virus), HAV (Hepatitis A-Virus), Vibrio Cholera-Antigen, Borrelia burgdorferi, Helicobacter pylori oder Malaria-Antigen.

Zu derartigen Wirksubstanzen im Sinne der vorliegenden Erfindung gehört auch die DNA eines Antiidiotyp-Antikörpers oder seiner Antigen-bindenden Fragmente, dessen Antigenbindungsstrukturen (die "complementary determining regions") Kopien der Protein- oder Kohlenhydratstruktur des Neutralisationsantigens des Infektionserregers darstellen (s.o.).

Derartige Antiidiotyp-Antikörper und ihre Spaltprodukte können besonders Kohlenhydratantigene bei bakteriellen Infektionserregern ersetzen und sind beispielsweise bei Hawkins et al. (J. Immunother. 14, 273 (1993)) und Westerink und Apicella (Springer Seminars in Immunopathol. 15, 227 (1993)) übersichtlich beschrieben.

Andere Effektoren sind beispielsweise Gene von "Tumorvakzinen". Hierzu gehören Antigene auf Tumorzellen, wie zum Beispiel von Sedlacek et al., Contrib. to Oncol. 32, Karger Verlag, München (1988) und Contrib. to Oncol 43, Karger Verlag, München (1992) übersichtlich dargestellt.

Weitere Beispiele stellen die Gene von folgenden Antigene bzw. von folgenden Antiidiotypantikörper dar: Sialyl Lewis; Peptide auf Tumoren, welche von T-Zellen erkannt werden; von Onkogenen exprimierte Proteine; Blutgruppenantigene und deren Vorläufer; Antigene auf dem polymorphen epithelialen Mucin; oder Antigene auf Heat Shock Proteinen.

Für die Therapie von chronischen Infektionserkrankungen dienen als Zielzellen beispielweise Leberzellen, Lymphozyten und/oder Makrophagen, Epithelzellen oder Endothelzellen, als Promotoren bespielsweise virusspezifische oder zellspezifische und zellzyklusspezifische Promotoren, und als Effektoren beispielsweise folgende Gene:

Gene, kodierend für ein Protein, welches zytostatische, apoptotische oder zytotoxische Wirkungen aufweist, oder kodierend für ein Enzym, welches eine Vorstufe einer antiviralen oder zytotoxischen Substanz in die aktive Substanz spaltet.

Gene kodierend für antivirale Proteine, wie antiviral wirksame Cytokine und Wachstumsfaktoren wie beispielsweise IFNα, IFNβ, IFN-γ, TNFβ, TNFα, IL-1 oder TGFβ, oder Antikörper einer Spezifität, die das jeweilige Virus inaktiviert oder dessen VH und VL enthaltende Fragmente oder dessen über einen Linker verbundene VH und VL Fragmente, wie bereits beschrieben. Antikörper gegen Virusantigene sind beispielsweise: anti HBV, anti HCV, anti HSV, anti HPV, anti HIV, anti EBV, anti HTLV, Anti Coxsackie Virus oder anti Hantaan Virus.

Ein anderes antiviral wirkendes Protein ist ein Rev bindendes Protein. Diese Proteine binden an die Rev-RNA und inhibieren Rev-abhängige posttranskriptionelle Stufen der Retrovirus-Genexpression. Beispiele für Rev-bindende Proteine sind: RBP9-27, RBP1-8U, RBP1-8D oder Pseudogene von RBP1-8.

Gene kodierend für antibakterielle Proteine, wie beispielsweise Antikörper, die bakterielle Toxine neutralisieren oder Bakterien opsonieren. Beispielsweise gehören hierzu Antikörper gegen Meningokokken C oder B, E. coli, Borrelia, Pseudomonas, Helicobacter pylori oder Staphylococcus aureus.

Insbesondere betrifft die vorliegende Erfindung die folgenden Gegenstände:
1. Einzelkettiges, mehrfach-antigenbindendes Molekül enthaltend folgende Komponenten:
   (a) eine variable Domäne einer schweren Kette eines Immunglobulins (VH) mit einer ersten Spezifität (A) und funktionelle Teile hiervon,
   (b) eine variable Domäne einer leichten Kette eines Immunglobulins (VL) mit einer zweiten Spezifität (B) und funktionelle Teile hiervon,
   (c) eine variable Domäne einer schweren Kette eines Immunoglobulins (VH) mit der Spezifität (B) und funktionelle Teile hiervon, sowie
   (d) eine variable Domäne einer leichten Kette eines Immunglobulins (VL) mit der Spezifität (A) und funktionelle Teile hiervon,
   wobei die Domäne VH und VL in Form eines VH-VL-Kontruktes oder VL-VH-Konstruktes verbunden sind, dadurch gekennzeichnet, daß die beiden VH-VL-Konstrukte über ein Peptid (P) verbunden sind.
2. Einzelkettiges, mehrfach-antigenbindendes Molekül nach Ziffer 1, dadurch gekennzeichnet, daß mehr als zwei VH-VL-Konstrukte enthalten sind.
3. Einzelkettiges, mehrfach-antigenbindendes Molekül nach Ziffer 1 oder 2,
   dadurch gekennzeichnet, daß die VH-VL-Konstrukte über Domänen mit gleicher Spezifität verbunden sind.
4. Einzelkettiges, mehrfach-antigenbindendes Molekül nach einer der Ziffern 1-3, dadurch gekennzeichnet, daß die Spezifitäten (A) und (B) im wesentlichen identisch sind.
5. Einzelkettiges, mehrfach-antigenbindendes Molekül nach einer der Ziffern 1-4, dadurch gekennzeichnet, daß die Domänen VH und VL über einen Peptid-Linker (L) in Form eines VH-L-VL-Konstuktes oder VL-L-VH-Konstruktes verbunden sind.
6. Einzelkettiges, mehrfach-antigenbindendes Molekül nach Ziffer 5, dadurch gekennzeichnet, daß der Linker (L) ca. 1-20 Aminosäuren, vorzugsweise ca. 1-5 Aminosäuren lang ist.
7. Einzelkettiges, mehrfach-antigenbindendes Molekül nach Anpruch 5, dadurch gekennzeichnet, daß der Linker (L) die Aminosäuresequenz GGGGS (SEQ ID NO: 7) enthält.
8. Einzelkettiges, mehrfach-antigenbindendes Molekül nach einer der Ziffern 1-7, dadurch gekennzeichnet, daß das Peptid (P) ca. 12-40 Aminosäuren, vorzugsweise ca. 12-20 Aminosäuren, insbesondere ca. 14 Aminosäuren lang ist.
9. Einzelkettiges, mehrfach-antigenbindendes Molekül nach Ziffer 8, dadurch gekennzeichnet, daß das Peptid (P) die Aminosäuresequenz GGGGSGGRASGGGS (SEQ ID NO: 1) oder GGGGSGGRASGGGGS (SEQ ID NO: 2) enthält.
10. Einzelkettiges, mehrfach-antigenbindendes Molekül nach einer der Ziffern 1-9, dadurch gekennzeichnet, daß das genannte Molekül als eine weitere Komponente einen oder mehrere Effektor/en (E) enthält.
11. Einzelkettiges, mehrfach-antigenbindendes Molekül nach Ziffer 10, dadurch gekennzeichnet, daß der Effektor (E) über ein Bindeglied (B) an das genannte Molekül gebunden ist.
12. Einzelkettiges, mehrfach-antigenbindendes Molekül nach Ziffer 11, dadurch gekennzeichnet, daß das Bindeglied (B) eine Protease-Spaltsequenz, vorzugsweise eine PSA-, Cathepsin-, Plasminogen- und/oder Plasminogenaktivator-Spaltsequenz enthält.
13. Einzelkettiges, mehrfach-antigenbindendes Molekül nach einer der Ziffern 1-12, dadurch gekennzeichnet, daß die erste Spezifität (A) gegen ein zu analysierendes Molekül gerichtet ist und die zweite Spezifität (B) gegen einen Analyten gerichtet ist.
14. Einzelkettiges, mehrfach-antigenbindendes Molekül nach Ziffer 13, dadurch gekennzeichnet, daß der Analyt ein radioaktives Molekül, ein fluoreszierendes Molekül und/oder ein Enzym ist.
15. Einzelkettiges, mehrfach-antigenbindendes Molekül nach einer der Ziffern 10-12, dadurch gekennzeichnet, daß die erste Spezifität (A) gegen ein zu analysierendes Molekül gerichtet ist, die zweite Spezifität (B) gegen ein anderes zu analysierendes Molekül gerichtet ist und der Effektor (E) ein Analyt ist.
16. Einzelkettiges, mehrfach-antigenbindendes Molekül nach Ziffer 15, dadurch gekennzeichnet, daß der Analyt ein radioaktives Molekül, ein fluoreszierendes Molekül und/oder ein Enzym ist.
17. Einzelkettiges, mehrfach-antigenbindendes Molekül nach einer der Ziffern 1-12, dadurch gekennzeichnet, daß das Peptid (P) und/oder der Effektor (E) ein fusiogenes Peptid enthält.
18. Einzelkettiges, mehrfach-antigenbindendes Molekül nach einer der Ziffern 1-17 dadurch gekennzeichnet, daß die erste Spezifität (A) gegen eine Zielzelle gerichtet ist und die zweite Spezifität (B) gegen einen Vektor.
19. Einzelkettiges, mehrfach-antigenbindendes Molekül nach Ziffer 18, dadurch gekennzeichnet, daß der Vektor eine Nukleinsäure, ein kationisches Peptid oder Protein, ein kationisches Lipid, ein kationisches Polymer, ein kationisches Porphyrin oder ein viraler Vektor ausgewählt aus der Gruppe enthaltend AdV-, AAV-, Vaccinia-, RSV-, HSV-, Influenza- oder Lentivirus-Vektor, ist.
20. Einzelkettiges, mehrfach-antigenbindendes Molekül nach einer der Ziffern 1-12, dadurch gekennzeichnet, daß die erste Spezifität (A) gegen eine Zellmembran, insbesondere gegen Lymphozyten, Makrophagen, Monozyten, Granulozyten, hämatopoetische Zellen, Endothelzellen, glatte Muskelzellen, quergestreifte Muskelzellen, Epithelzellen, Leberzellen, Nierenzellen, Gliazellen, Zellen des Stützgewebes, Tumorzellen oder Leukämiezellen, oder gegen Proteine der extrazellulären Matrix, des Komplementsystems, des Gerinnungssystems, des Kininsystems, des Blutplasmas, des Stützgewebes, oder gegen Cytokine oder Chemokine, oder gegen körpereigene oder körperfremde Toxine oder gegen Arzneimittel, insbesondere Digitalis, und/oder gegen Infektionserreger, wie insbesondere bakterielle, virale und/oder parasitäre Infektionserreger, gerichtet ist.
21. Einzelkettiges, mehrfach-antigenbindendes Molekül nach einer der Ziffern 1-12 und 20, dadurch gekennzeichnet, daß die zweite Spezifität (B) gegen eine Zellmembran, insbesondere gegen Lymphozyten, Makrophagen, Monozyten oder Granulozyten, gegen Cytokine, Chemokine oder Wachstumsfaktoren, gegen Proteine des Komplementsystems, gegen Proteine des Gerinnungssystems, gegen fibrinolytische Proteine, gegen Enzyme, welche an der Zielstruktur die unwirksame Vorstufe eines Wirkstoffes in einem aktiven, insbesondere zytotoxischen Wirkstoff überführen können, gegen Peptidhormone oder Steroidhormone, gegen den konstanten Teil eines Immunglobulins, gegen einen Mediator, wie insbesondere Histamin, Serotonin, Leukotrien, Prostacyclin oder Kinin, gegen Infektionserreger, gegen körpereigene oder körperfremde Toxine, gegen Arzneimittel, insbesondere Digitalis, gerichtet ist.
22. Einzelkettiges, mehrfach-antigenbindendes Molekül nach einer der Ziffern 1-12, 20 und 21, dadurch gekennzeichnet, daß der Effektor (E) ausgewählt ist aus einer Transmembrandomäne, einem Glykophospholipidanker, dem Liganden bindenden Teil eines Rezeptors, einem Liganden für einen Rezeptor oder der den Rezeptor bindenden Teilsequenz des Liganden, einem Peptidhormon, einem Cytokin, einem Wachstumsfaktor, einem Wachstumsfäktorinhibitor, einem Chemokin, einem Interferon, einem Mediator, einem kreislaufwirksamen Peptid, einem Enzym, welches eine inaktive Vorstufe eines Wirkstoffes in einen aktiven Wirkstoff überführt; einem Protein, welches die Gerinnung aktiviert oder inhibiert; einem Protein, welches die Fibrinolyse aktiviert oder inhibiert; einem Protein, welches das Komplementsystem aktiviert oder inhibiert; einer oder mehreren konstanten Domänen eines Immunglobulins; einem zytotoxischen Peptid; einem anderen, einzelkettigen, einfach oder mehrfach-, insbesondere zweifach-antigenbindenden Molekül; einem Tumorantigen oder einem Antigen eines Infektionserregers, wie beispielsweise einem bakteriellen Antigen oder einem viralen Antigen; einem Peptid enthaltend Cystein und/oder einem di- bzw. multimerisierenden Peptid.
23. Nukleinsäure kodierend für ein einzelkettiges, mehrfach-antigenbindendes Molekül nach einer der Ziffern 1-22.
24. Nukleinsäure nach Ziffer 23, dadurch gekennzeichnet, daß die genannte Nukleinsäure am 5'-Ende eine Nukleotidsequenz kodierend für eine Signal- oder Transmembransequenz enthält.
25. Nukleinsäure nach Ziffer 23 oder 24, dadurch gekennzeichnet, daß die genannte Nukleinsäure am 5'-Ende einen Promoter und/oder Aktivator enthält.
26. Nukleinsäure nach Ziffer 25, dadurch gekennzeichnet, daß der Aktivator zellspezifisch, zellzyklusspezifisch, metabolisch-spezifisch und/oder durch einen Wirkstoff aktivierbar oder suprimierbar ist.
27. Nukleinsäure nach einer der Ziffern 23-26, dadurch gekennzeichnet, daß die genannte Nukleinsäure am 5'-Ende des Startkodons die Sequenz GCCACC oder GCCGCC enthält.
28. Vektor enthaltend eine Nukleinsäure nach einer der Ziffern 23-27.
29. Vektor nach Ziffer 28, dadurch gekennzeichnet, daß der Vektor ein viraler oder nicht-viraler Vektor ist.
30. Vektor nach Ziffer 29, dadurch gekennzeichnet, daß der nicht-virale Vektor ausgewählt ist aus einem kationischen Lipid, einem kationischen Polymer, einem kationischen Peptid oder einem kationischen Porphyrin.
31. Zelle enthaltend eine Nukleinsäure nach einer der Ziffern 23-27 oder einen Vektor nach Ziffer 28 oder 29.
32. Zelle nach Ziffer 31, dadurch gekennzeichnet, daß die Zelle eine Bakterien-, Hefe-, Insekten- oder Säugerzelle ist.
33. Zelle nach Ziffer 32, dadurch gekennzeichnet, daß die Säugerzelle ein Lymphozyt, ein Makrophage, eine Gliazelle, eine Epithelzelle, eine Leberzelle, eine Nierenzelle, eine Knochenmarkszelle, eine Endothelzelle, eine glatte oder quergestreifte Muskelzelle oder ein Fibroblast ist.
34. Verfahren zur Herstellung eines einzelkettigen, mehrfach-antigengebundenen Moleküls, dadurch gekennzeichnet, daß eine Zelle nach Ziffer 31 oder 32 kultiviert und das Expressionsprodukt gegebenenfalls isoliert wird.
35. Arzneimittel enthaltend ein einzelkettiges, mehrfach-antigenbindendes Molekül nach einer der Ziffern 1-12 und 17-22, eine Nukleinsäure nach einer der Ziffern 23-27, einen Vektor nach einer der Ziffern 28-30 oder eine Zelle nach einer der Ziffern 31-33.
36. Diagnostikum enthaltend ein einzelkettiges, mehrfach-antigenbindendes Molekül nach einer der Ziffern 13-16.
37. Verwendung eines einzelkettigen, mehrfach-antigenbindenden Moleküls nach einer der Ziffern 1-22, einer Nukleinsäure nach einer der Ziffern 23-27, eines Vektors nach einer der Ziffern 28-30 oder einer Zelle nach einer der Ziffern 31-33 zur Therapie, Prophylaxe oder Diagnose von Tumorerkrankungen, Autoimmunerkrankungen, Entzündungserkrankungen, Erkrankungen des Blutes, insbesondere des Blutgerinnungs- und/oder Blutkreislaufsystems, Erkrankungen des Nervensystems und/oder Infektionserkrankungen.

Die folgenden Figuren und Beispiele sollen die Erfindung näher erläutern, ohne sie darauf zu beschränken.

### Beschreibung der Figuren

Fig. 1 beschreibt schematisch den Aufbau eines einzelkettigen, zweifach-antigenbindenden Moleküls
Fig. 2 beschreibt schematisch ein einzelkettiges, zweifach-antigenbindendes Molekül mit Effektor.

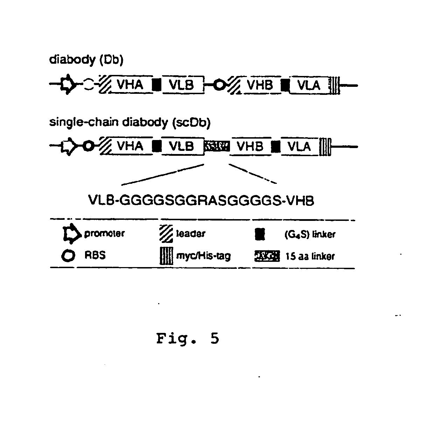

Fig. 3 und Fig. 5 beschreiben schematisch Nukleinsäurekonstrukte kodierend für ein einzelkettiges, zweifach-antigenbindendes Molekül
Fig. 4 beschreibt schematisch ein Nukleinsäurekonstrukt kodierend für ein einzelkettiges, zweifach-antigenbindendes Molekül mit Effektor.
Fig. 6 zeigt die Rekrutierung der β-Galaktosidase an plastikgebundenem CEA durch das erfindungsgemäße einzelkettige, zweifach-antigenbindende Protein (scDb-CEAGal) im Vergleich zu Diabodies gegen CEA und E. coli β-Galaktosidase (CEAGal). Als negative Kontrolle wude eine Mikrotiterplatte mit BSA beschichtet.
Fig. 7 zeigt die Rekrintierung der β-Galaktosidase durch scDb-CEAGal, welches von Säugerzellen sekretiert wurde, wobei verschiedene Mengen an scDb-CEAGal und β-Galaktosidase in CEA-beschichteten Mikrotiterplatten eingesetzt wurden. Als Substrat wurde o-Nitrophenyl-β-D-galactopyranosid eingesetzt. Als negative Kontrolle wurde eine Mikrotiterplatte mit BSA beschichtet.
Fig. 8A zeigt das Absterben von LoVo-Zellen in Anwesenheit des erfindungsgemäßen einzelkettigen, zweifach-antigenbindenden Proteins (scDb), β-Galaktosidase (β-Gal), Daunomycin-β-D-galaktopyranosid (prodrug) und/oder Daunomycin (drug).
Fig. 8B zeigt Kontrollexperimente mit einer CEA-negativen Zellinie (A549).

### Beispiele:

### Beispiel 1

Herstellung und bakterielle Expression eines einzelkettigen, zweifach-antigenbindenden Proteins

Die Herstellung eines einzelkettigen, zweifach-antigenbindenden Proteins wird am Beispiel eines Proteins, das die Antigene "Carcinoembryonic Antigen" (CEA) und E. coli β-Galaktosidase erkennt, dargestellt:

Folgende DNA-Sequenzen wurden in Richtung 5' zu 3' wie folgt zusammengefügt:
- LacZ-Promotor
- bakterielle, ribosomale Bindestruktur (AAGGAG)
- bakterielle Signalsequenz pelB (Power et al., Gene 113, 95-99 (1992))
- VH-anti-CEA (Kontermann et al., Immunotechnol. 3, 137 (1997))
- Linker GGGS (SEQ ID NO: 8; Kontermann et al., (1997))
- VL-anti-β-Galaktosidase (Kontermann et al., (1997))
- verbindendes Peptid GGGGSGGRASGGGGS (SEQ ID NO: 1)
- VH-anti-β-Galaktosidase (Kontermann et al., (1997))
- Linker GGGGS (SEQ ID NO: 7)
- VL-anti-CEA (Kontermann et al., (1997))
- Myc-Epitop für Antikörper 9E10 EQKLISEEDLN (SEQ ID NO: 9; Munro & Pelham, Cell 46, 291-300 (1986))
- Polyhistidin HHHHHH (SEQ ID NO: 10) zur Reinigung mittels IMAC (Kontermann et al., (1997))

Die Verknüpfung des Konstrukts wurde über geeignete Restriktionsstellen, die über PCR-Amplifikation an den Termini der verschiedenen DNA-Sequenzen mitgeführt wurden, ermöglicht (Kontermann et al., Immunotechnol. 3, 137 (1997)).

Die Verknüpfung erfolgte mit Hilfe von dem Fachmann bekannten, für die Restriktionsstellen spezifischen Enzyme und DNA-Ligasen. Die Enzyme sind käuflich zu erwerben. Das Konstrukt wurde in den bakteriellen Expressionsvektor pAB1 (Kontermann et al., (1997)) einkloniert.

Für die Klonierung wurden die Oligonukleotid-Primer LMB2 und LMB3 (Kontermann, R.E. (1997), supra) sowie
Vκ-NotForκ: 5'-TTG TTC TGC GGC CGC CCG TTT CAG CTG CAG CTT GGT GCC AGC ACC-3' (SEQ ID NO: 11),
scDb-AscBack: 5'-TGC ATG CTA GGG CGC GCC TCG GGC GGA GGT GGC TCA CAG GTG CAG CTG GTG CAA TCT GG-3' (SEQ ID NO: 12),
scDb-AscFor: GCT CGG TAA GGC GCG CCC AGC GCT GCC ACC GCC TCC ACC TAG GAC GGT CAG CTT GGT CCC-3' (SEQ ID NO: 13) und
pelB-Metminus: 5'-TTA CTC GCG GCC CAG CCG GCC ACG GCC CAG GT-3' (SEQ ID NO: 14) verwendet.

Hierzu wurden aus dem Diabody CEAGal (Kontermann, R.E. (1997)) mit Hilfe der Primer LMB2 und LMB3 die Fragmente VHB-VLA (Fragment 1) und VHA-VLB (Fragment 2) amplifiziert und Gel-gereinigt. Fragment 1 wurde anschließend mit den Primern Vκ-NotFor und scDb-AscBack amplifiziert, um eine AscI-Stelle und 7 Aminosäuren des Linkers einzuführen. Fragment 2 wurde mit den Primern LMB3 und scDb-AscFor λ amplifiziert, um eine AscI-Stelle und 8 Aminosäuren des Linkers einzuführen. Die Fragmente wurden mit Ascl und NotI (Fragment 1) und SfiI und AscI (Fragment 2) hydrolysiert und in den bakteriellen Expressionsvektor pAB1 (Kontermann, R.E. (1997)) kloniert. Das resultierende Insert kodiert ein einzelkettiges Polypeptid, worin VHA-VLB über einen 15 aminosäurelangen Linker mit der Sequenz GGGGSGGRASGGGGS (SEQ ID NO: 2) an VHB-VLA gebunden ist.

Das Plasmid wurde anschließend in TG1-Bakterien eingeführt und diese mit den dem Fachmann geläufigen Methoden kultiviert (Kontermann et al., (1997)).

Die Bakterien wurden aufgeschlossen und das einzelkettige, zweifach-antigenbindende Protein durch immobilisierte Metallaffinitätschromatographie (IMAC) (Hochuli et al., Bio/Techn. 6, 1321-1325 (1988)) aus der periplasmatischen Präparation gereinigt. Details dieser Methode sind bei Kontermann et al., (1997) beschrieben.

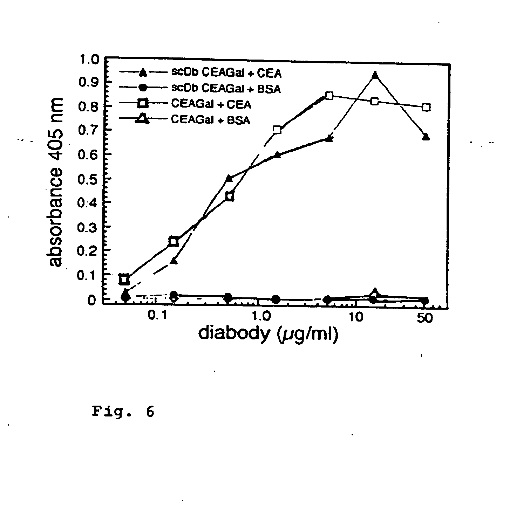

Das gereinigte Protein hat ein Molekulargewicht von 60 kDa, wie durch SDS-Polyacrylamidgelelektrophorese und Gelfiltration gezeigt wurde. Etwa 200-300 µg dieses Moleküls konnten pro Liter Bakterienkultur gereinigt werden. Das bakteriell exprimierte, einzelkettige, zweifach-antigenbindende Protein reagierte im ELISA mit CEA sowie β-Galaktosidase und war in der Lage, das Enzym zu plastikgebundenem CEA zu rekrutieren, wie anhand der Konversion von o-Nitrophenyl-β-D-galaktopyranosid gezeigt werden konnte (Fig. 6). Weiterhin konnte dieses einzelkettige, zweifach-antigenbindende Protein lebende wie auch fixierte CEA-exprimierende LoVo-Zellen durch Rekrutierung von β-Galaktosidase und Umsatz des Substrats 5-Bromo-4-chloro-3-indolyl-β-D-galactopyranosid (X-Gal) anfärben. Hierzu wurden LoVo-Zellen mit 10 µg/ml des entsprechenden antigenbindenden Proteins mit 10 µg/ml β-Galaktosidase und X-Gal (0,8 mg/ml in PBS, 3mM-Kaliumeisen(III)-cyanid 3mM Kaliumeisen(II)-cyanid) inkubiert. Keine Färbung war mit verschiedenen Kontrollzellen (A549, HEK293) bzw. mit einem Diabody gegen Hühnereilysozym und β-Galaktosidase (HELGal; Kontermann et al., (1997)) zu beobachten.

### Beispiel 2

### Eukaryotische Expression eines einzelkettigen, zweifach-antigenbindenden Proteins

Für die Expression in eukaryotischen Zellen wurde die kodierende Region des einzelkettigen, zweifach-antigenbindenden Proteins in einen eukaryotischen Expressionsvektor kloniert (pSecTagA, Invitrogen), wobei die bakterielle Signalsequenz durch die Ig-κ-Signalsequenz, die bereits im Vektor enthalten ist, ersetzt wurde.

Hierzu wurde das einzelkettige Konstrukt mit den Primern LMB2 und pelB-Metminus amplifiziert, wodurch Methionin des pelB-Leaders (Position 21) durch Threonin ersetzt wurde. Anschließend wurde mit SfiI und EcoRI hydrolysiert und in den Vektor pSecTagA kloniert. Das reife einzelkettige antigenbindende Protein enthält 7 zusätzliche Aminosäuren (AAQPATA; SEQ ID NO: 15) am N-Terminus der VHA-Domäne.

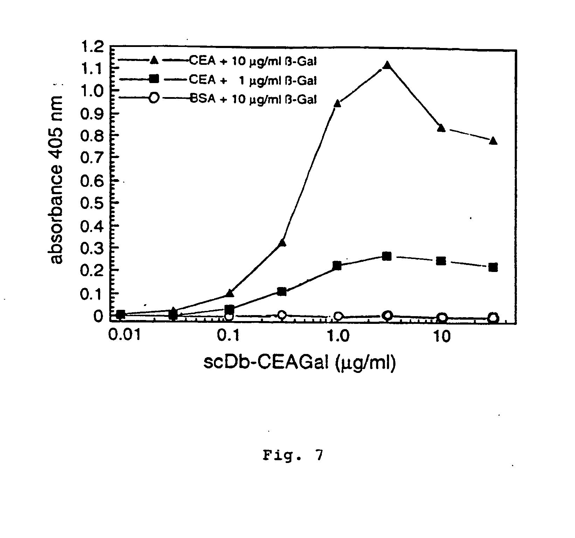

Das Plasmid wurde mit Lipofectamin (Gibco) transient in eukaryotischen HEK 293 Zellen transfiziert. Stabile Zellen wurden in Gegenwart von Zeocin selektiert. Mit diesen Zellen konnte in Immunofluoreszenzexperimenten eine Anfärbung des endoplasmatischen Retikulums ER und des Golgi-Apparates nachgewiesen werden. Ferner konnte die Sekretion des einzelkettigen, zweifach-antigenbindenden Proteins durch Immunpräzipitation eines 60 kDa Proteins aus dem Überstand 35 S-Metmarkierter Zellen sowie durch Reinigung mittels immobilisierter Metallaffinitätschromatographie (IMAC) nachgewiesen werden. Das gereinigte Protein ist funktionell in der Bindung von β-Galaktosidase und CEA-beschichteten Mikrotiterplatten bzw. der Rekrutierung von β-Galaktosidase zu plastikgebundenem CEA aktiv (Fig. 7).

### Beispiel 3

### In vitro Enzymrekruderung durch Kokultivierung mit einzelkettigen, zweifachantigenbindendem Protein sezernierenden Zellen

Durch Kokultivierung von das einzelkettige, zweifach-antigenbindende Protein produzierenden HEK 293-Zellen und CEA-positiven LoVo-Zellen wurde die Rekrutierung in vitro untersucht. Dazu wurden zunächst die das erfindungsgemäße Protein produzierenden Zellen mit den LoVo-Zellen in Transwell-Zellkulturschalen (Costar), bei denen die beiden Zellinien durch eine Membran getrennt sind, kultiviert. Nach zwei Tagen wurde β-Galaktosidase (10 µg/ml) dazugegeben und die Rekrutierung durch Zugabe des Substrates X-Gal nachgewiesen. Eine spezifische Färbung war bei Kokultivierung mit den das einzelkettige, zweifach-antigenbindende Protein produzierenden Zellen festzustellen, während Kontrollexperimente mit nicht transfizierten HEK 293-Zellen keine Färbung zeigten.

Ebenfalls negativ waren Experimente, bei denen die LoVo-Zellen durch A549-Zellen (CEA-negativ) ersetzt wurden, bzw. Experimente, bei denen die das einzelkettige, zweifach-antigenbindende Protein produzierenden Zellen mit Enzym und Substrat inkubiert wurden. Letzteres verdeutlicht, daß die das einzelkettige, zweifach-antigenbindende Protein produzierende Zellen selbst nicht zur Bindung des Enzyms in der Lage sind.

In einem weiteren Experiment wurden die das einzelkettige, zweifach-antigenbindende Protein sekretierenden HEK 293-Zellen und die LoVo-Zellen direkt mit einem Inokulationsverhältnis von 1:4 bis 1:24 kokultiviert. Um die HEK 293-Zellen von den LoVo-Zellen zu unterscheiden, wurden erstere vor Zugabe der LoVo-Zellen mit CM-DiI (Molecular Probes) gefärbt (rote Fluoreszenz). Nach zwei Tagen wurde β-Galaktosidase zugegeben und die Bindung an Zellen durch Zugabe von X-Gal (10 µg/ml) bzw. durch indirekte Immunfluoreszenz mit einem anti-β-Galaktosidase Antikörper (Biotrend) nachgewiesen. Auch in diesen Experimenten zeigt sich eine spezifische Rekrutierung des Enzyms zu den LoVo-Zellen, während die HEK 293-Zellen nicht gefärbt wurden. Negativ waren Kontrollexperimente mit nicht-transfizierten HEK 293-Zellen. Diese Experimente belegen, daß das einzelkettige, zweifach-antigenbindende Protein spezifisch und selektiv Tumorzellen erkennt. -

In weiteren Experimenten wurde die Konvertierung von nicht-toxischem Daunomycin-β-D-galaktopyranosid in das zytotoxische Daunomycin untersucht. Durch einstündige Inkubation von LoVo-Zellen mit gereinigtem, einzelkettigen, zweifach-antigenbindenden Protein (10 µg/ml) sowie die anschließende einstündige Inkubation bei 37° mit β-Galaktosidase (1 µg/ml) und Daunomycin-β-D-galaktopyranosid (2 µM) konnte eine spezifische Kernlokalisation des entstehenden Daunomycins mittels Autofluoreszenz der Substanz nachgewiesen werden, vergleichbar der Färbung durch direkte Inkubation mit Daunomycin. Keine Kernfärbung war in Abwesenheit von einzelkettigem, zweifach-antigenbindenden Protein bzw. β-Galaktosidase sowie mit Kontrollzellen (HEK 293; einzelkettiges, zweifach-antigenbindendes Protein produzierende HEK 293) festzustellen. Diese Experimente belegen, daß das einzelkettige, zweifach-antigenbindende Protein in der Lage ist, ein Enzym an eine Tumorzelle zu rekrutieren und daß dieses Enzym zur Konvertierung einer nicht toxischen Vorstufe in eine toxische Substanz eingesetzt werden kann.

In weiteren Experimenten kann dieser Effekt dazu benutzt werden, Tumorzellen spezifisch abzutöten.

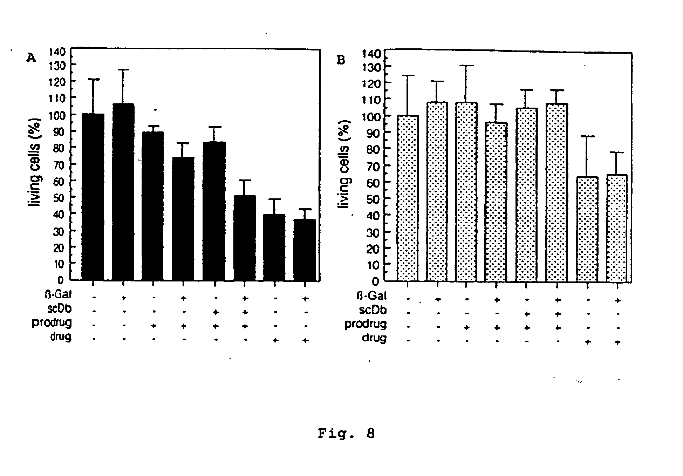

Hierzu wurden die LoVo-Zellen in 96-well Platten mit dem einzelkettigen, zweifach-antigenbindenden Protein (10 µg/ml) und anschließend mit Daunomycin-β-D-galaktopyranosid (5 µM) eine Stunde lang bei 37°C inkubiert. Das Absterben der Zellen wurde nach 2 Tagen mittels eines WST-Tests (Boehringer Mannheim) analysiert (Fig. 8). Hierbei war das Absterben der Zellen bei der Umwandlung des Prodrug in das Drug annähernd genausogut wie in Anwesenheit des Drug (Fig. 8A). In Abwesenheit einer Komponente oder mit CEA-negativen A549-Zellen wurde im wesentlichen keine Wirkung festgestellt (Fig 8B).

### Beispiel 4

### Herstellung von Konstrukten zur intrazellulären Expression von einzelkettigen, zweifach-antigenbindenden Proteinen

Für die intrazelluläre Expression des einzelkettigen, zweifach-antigenbindenden Proteins wird die DNA des Gens für das einzelkettige, zweifach-antigenbindende Protein mit unterschiedlichen Primern amplifiziert:
- transmembranes einzelkettiges, zweifach-antigenbindendes Protein (TM-scDAP). Hierfür wird ein Primer verwendet, der am 3'-Ende des Gens die Transmembrandomäne von PDGFR einfügt
- (LPFKVVVISAIIALVVLTIISLIILIMLWQKKPRYES; SEQ ID NO: 16).
- Endoplasmatisches Retikulum lokalisiertes einzelkettiges (single chain), zweifach(double)-antigenbindendes Protein (ER-scDAP). Hierfür wird ein Primer verwendet, der am 3'-Ende des Gens ein ER-Retentionssignal einfügt (SEKDEL; SEQ ID NO:-18).
- zytoplasmatisch lokalisiertes einzelkettiges, zweifach-antigenbindendes Protein (cyto-scDAP). Hierfür wird ein Primer benutzt, der am 5'-Ende des Gens die Signalsequenz durch ein Methionin und eine Kozaksequenz zur optimalen Translationsinitiation ersetzt.
- kernlokalisiertes einzelkettiges, zweifach-antigenbindendes Protein (nuc-scDb). Hierfür werden Primer benutzt, die am 5'-Ende des Gens die Signalsequenz durch ein Methionin und eine Kozaksequenz zur optimalen Translationsinitiation ersetzen und am 3'-Ende des Gens eine Kernlokalisierungssequenz einfügen (PKKKRKVGGGT; SEQ ID NO: 17; die Kernlokalisierungssequenz ist unterstrichen).

Diese Fragmente werden in geeigneten eukaryotischen Expressionsvektoren kloniert (pSecTagA bzw. pcDNA3; Invitrogen). Die resultierenden Konstrukte (TM-scDAP, ER-scDAP, cyto-scDAP, nuc-scDAP, sowie sec-scDAP (= sezerniertes Protein; siehe Beispiel 2) werden anschliessend in eukaryotische Zellen (3T3) transient transfiziert und die Lokalisierung des exprimierten Proteins durch Immunfluoreszenz mit Hilfe eines anti-Myc-Epitop-Antikörpers untersucht. Für die Konstrukte sec-scDAP, ER-scDAP und TM-scDAP kann eine Färbung, wie sie für den sekretorischen Weg typisch ist, nachgewiesen werden, während cyto-scDAP eine diffuse Lokalisierung im Zytoplasma und nuc-scDAP eine Kernlokalisierung zeigt.

## Patentansprüche

1. Einzelkettiges, mehrfach-antigenbindendes Molekül enthaltend folgende Komponenten:
(a) eine variable Domäne einer schweren Kette eines Immunglobulins (VH) mit einer ersten Spezifität (A) und funktionelle Teile hiervon,
(b) eine variable Domäne einer leichten Kette eines Immunglobulins (VL) mit einer zweiten Spezifität (B) und funktionelle Teile hiervon,
(c) eine variable Domäne einer schweren Kette eines Immunoglobulins (VH) mit der Spezifität (B) und funktionelle Teile hiervon, sowie
(d) eine variable Domäne einer leichten Kette eines Immunglobulins (VL) mit der Spezifität (A) und funktionelle Teile hiervon,
wobei die Domänen VH und VL in Form eines VH-VL-Kontruktes oder VL-VH-Konstruktes verbunden sind, **dadurch gekennzeichnet, daß** die beiden VH-VL- oder die beiden VL-VH-Konstrukte über ein Peptid (P) verbunden sind und die VH-VL- oder VL-VH-Konstrukte über Domänen mit gleicher Spezifität verbunden sind, und die einzelnen Spezifitäten (A) und (B) im wesentlichen identisch zur molekularen Struktur einer Immunglobulin-Domäne der besagten Spezifität sind.

2. Einzelkettiges, mehrfach-antigenbindendes Molekül nach Anspruch 1, **dadurch gekennzeichnet, daß** die Domänen VH und VL über einen Peptid-Linker (L) in Form eines VH-L-VL-Konstuktes oder VL-L-VH-Konstruktes verbunden sind.

3. Einzelkettiges, mehrfach-antigenbindendes Molekül nach Anspruch 2, **dadurch gekennzeichnet, daß** der Linker (L) ca. 1-20 Aminosäuren, vorzugsweise ca. 1-5 Aminosäuren lang ist.

4. Einzelkettiges, mehrfach-antigenbindendes Molekül nach einem der Ansprüche 1-3, **dadurch gekennzeichnet, daß** das Peptid (P) ca. 12-40 Aminosäuren, vorzugsweise ca. 12-20 Aminosäuren, insbesondere ca. 14 Aminosäuren lang ist.

5. Einzelkettiges, mehrfach-antigenbindendes Molekül nach einem der Ansprüche 1-4,
**dadurch gekennzeichnet, daß** das genannte Molekül als eine weitere Komponente einen oder mehrere Effektor/en (E) enthält.

6. Einzelkettiges, mehrfach-antigenbindendes Molekül nach einem der Ansprüche 1-5, **dadurch gekennzeichnet, daß** die erste Spezifität (A) gegen ein zu analysierendes Molekül gerichtet ist und die zweite Spezifität (B) gegen einen Analyten gerichtet ist.

7. Einzelkettiges, mehrfach-antigenbindendes Molekül nach Anspruch 6, **dadurch gekennzeichnet, daß** die erste Spezifität (A) gegen ein zu analysierendes Molekül gerichtet ist, die zweite Spezifität (B) gegen ein anderes zu analysierendes Molekül gerichtet ist und der Effektor (E) ein Analyt ist.

8. Einzelkettiges, mehrfach-antigenbindendes Molekül nach einem der Ansprüche 1-7 **dadurch gekennzeichnet, daß** die erste Spezifität (A) gegen eine Zielzelle gerichtet ist und die zweite Spezifität (B) gegen einen Vektor.

9. Einzelkettiges, mehrfach-antigenbindendes Molekül nach Anspruch 8, **dadurch gekennzeichnet, daß** der Vektor eine Nukleinsäure, ein kationisches Peptid oder Protein, ein kationisches Lipid, ein kationisches Polymer, ein kationisches Porphyrin oder ein viraler Vektor ausgewählt aus der Gruppe enthaltend AdV-, AAV-, Vaccinia-, RSV-, HSV-, Influenza- oder Lentivirus-Vektor, ist.

10. Einzelkettiges, mehrfach-antigenbindendes Molekül nach einem der Ansprüche 1-5, **dadurch gekennzeichnet, daß** die erste Spezifität (A) gegen eine Zellmembran, insbesondere gegen Lymphozyten, Makrophagen, Monozyten, Granulozyten, hämatopoetische Zellen, Endothelzellen, glatte Muskelzellen, quergestreifte Muskelzellen, Epithelzellen, Leberzellen, Nierenzellen, Gliazellen, Zellen des Stützgewebes, Tumorzellen oder Leukämiezellen, oder gegen Proteine der extrazellulären Matrix, des Komplementsystems, des Gerinnungssystems, des Kininsystems, des Blutplasmas, des Stützgewebes, oder gegen Cytokine oder Chemokine, oder gegen körpereigene oder körperfremde Toxine oder gegen Arzneimittel, insbesondere Digitalis, und/oder gegen Infektionserreger, wie insbesondere bakterielle, virale und/oder parasitäre Infektionserreger, gerichtet ist.

11. Einzelkettiges, mehrfach-antigenbindendes Molekül nach einem der Ansprüche 1-5 und 10, **dadurch gekennzeichnet, daß** die zweite Spezifität (B) gegen eine Zellmembran, insbesondere gegen Lymphozyten, Makrophagen, Monozyten oder Granulozyten, gegen Cytokine, Chemokine oder Wachstumsfaktoren, gegen Proteine des Komplementsystems, gegen Proteine des Gerinnungssystems, gegen fibrinolytische Proteine, gegen Enzyme, welche an der Zielstruktur die unwirksame Vorstufe eines Wirkstoffes in einem aktiven, insbesondere zytotoxischen Wirkstoff überführen können, gegen Peptidhormone oder Steroidhormone, gegen den konstanten Teil eines Immunglobulins, gegen einen Mediator, wie insbesondere Histamin, Serotonin, Leukotrien, Prostacyclin oder Kinin, gegen Infektionserreger, gegen körpereigene oder körperfremde Toxine, gegen Arzneimittel, insbesondere Digitalis, gerichtet ist.

12. Nukleinsäure kodierend für ein einzelkettiges, mehrfach-antigenbindendes Molekül nach einem der Ansprüche 1-11.

13. Vektor enthaltend eine Nukleinsäure nach Anspruch 12.

14. Zelle enthaltend eine Nukleinsäure nach Anspruch 12 oder einen Vektor nach Anspruch 13.

15. Verfahren zur Herstellung eines einzelkettigen, mehrfach-antigengebundenen Moleküls, **dadurch gekennzeichnet, daß** eine Zelle nach Anspruch 14 kultiviert und das Expressionsprodukt gegebenenfalls isoliert wird.

16. Arzneimittel enthaltend ein einzelkettiges, mehrfach-antigenbindendes Molekül nach einem der Ansprüche 1-5 und 8-11, eine Nukleinsäure nach Anspruch 12, einen Vektor nach Anspruch 13 oder eine Zelle nach Anspruch 14.

17. Diagnostikum enthaltend ein einzelkettiges, mehrfach-antigenbindendes Molekül nach einem der Ansprüche 6 oder 7.

18. Ein einzelkettiges, mehrfach-antigenbindendes Moleküls nach einem der Ansprüche 1-11, eine Nukleinsäure nach Anspruch 12, ein Vektors nach Anspruch 13 oder eine Zelle nach Anspruch 14 zur Verwendung in der Therapie, Prophylaxe oder Diagnose von Tumorerkrankungen, Autoimmunerkrankungen, Entzündungserkrankungen, Erkrankungen des Blutes, insbesondere des Blutgerinnungs- und/oder Blutkreislaufsystems, Erkrankungen des Nervensystems und/oder Infektionserkrankungen.

## Claims

1. A single-chain, multiple antigen-binding molecule comprising the following components:
(a) a variable domain of a heavy chain of an immunoglobulin (VH) with a first specificity (A), and functional parts thereof,
(b) a variable domain of a light chain of an immunoglobulin (VL) with a second specificity (B), and functional parts thereof,
(c) a variable domain of a heavy chain of an immunoglobulin (VH) with the specificity (B), and functional parts thereof, and
(d) a variable domain of a light chain of an immunoglobulin (VL) with the specificity (A), and functional parts thereof,
wherein the domain VH and VL are connected in form of a VH-VL construct or a VL-VH construct **characterized in that** the two VH-VL or the two VL-VH constructs are connected via a peptide (P) and the VH-VL constructs or VL-VH constructs are connected via domains with the same specificity and the individual specificities (A) and (B) are essentially identical to the molecular structure of an immunoglobulin domain of said specificity.

2. The single-chain, multiple antigen-binding molecule of claim 1, **characterized in that** the VH and VL domains are connected via a peptide linker (L) in the form of a VH-L-VL construct or VL-L-VH construct.

3. The single-chain multiple antigen-binding molecule of claim 2, **characterized in that** the linker (L) is about 1-20 amino acids, preferably about 1-5 amino acids, in length.

4. The single-chain multiple antigen-binding molecule of any one of claims 1-3, **characterized in that** the peptide (P) is about 12-40 amino acids, preferably about 12-20 amino acids, most preferably about 14 amino acids in length.

5. The single-chain multiple antigen-binding molecule of any one of claims 1-4, **characterized in that** said molecule comprises as a further component one or more effector(s) (E).

6. The single-chain multiple antigen-binding molecule of any one of claims 1-5, **characterized in that** the first specificity (A) is directed against a molecule to be analysed, and the second specificity (B) is directed against an analyte.

7. The single-chain multiple antigen-binding molecule of claim 6, **characterized in that** the first specificity (A) is directed against a molecule to be analysed, the second specificity (B) is directed against another molecule to be analysed, and the effector (E) is an analyte.

8. The single-chain multiple antigen-binding molecule of any one of claims 1-7, **characterized in that** the first specificity (A) is directed against a target cell, and the second specificity (B) is directed against a vector.

9. The single-chain multiple antigen-binding molecule of claim 8, **characterized in that** the vector is a nucleic acid, a cationic peptide or protein, a cationic lipid, a cationic polymer, a cationic porphyrin or a viral vector selected from the group comprising an AdV-, AAV-, vaccinia-, RSV-, HSV-, influenza- or lentivirus-vector.

10. The single-chain multiple antigen-binding molecule of any one of claims 1-5, **characterized in that** the first specificity (A) is directed against a cell membrane, particularly against lymphocytes, macrophages, monocytes, granulocytes, hematopoietic cells, endothelial cells, smooth muscle cells, striated muscle cells, epithelial cells, liver cells, kidney cells, glia cells, cells of the supporting tissue, tumor cells or leukemia cells, or against proteins of the extracellular matrix, of the complement system, of the coagulation system, of the kinin system, of the blood plasma, of the supporting tissue, or against cytokines or chemokines, or against endogenous or exogenous toxins, or against pharmaceuticals, in particular digitalis, and/or against infectious agents such as in particular bacterial-, viral-, and/or parasitic-infectious agents.

11. The single-chain multiple antigen-binding molecule of any one of claims 1-5 and 10, **characterized in that** the second specificity (B) is directed against a cell membrane particularly against lymphocytes, macrophages, monocytes, or granulocytes, against cytokines, chemokines or growth factors, against proteins of the complement system, against proteins of the coagulation system, against fibrinolytic proteins, against enzymes that are able to convert the inactive precursor of a drug into an active drug in particular into a cytotoxic drug on the target structure, against peptide hormones or steroid hormones, against the constant part of an immunoglobulin, against a mediator, in particular histamine, serotonin, leukotriene, prostacyclin or kinin, against infectious agents, against endogenous or exogenous toxins, against pharmaceuticals, in particular digitalis.

12. A nucleic acid coding for a single-chain multiple antigen-binding molecule according to any one of claims 1-11.

13. A vector comprising a nucleic acid according to claim 12.

14. A cell comprising a nucleic acid according to claim 12 or a vector according to claim 13.

15. A method for producing a single-chain multiple antigen-binding molecule, **characterized in that** a cell according to claim 14 is cultivated and the expression product is isolated where appropriate.

16. A pharmaceutical comprising a single-chain multiple antigen-binding molecule according to any one of claims 1-5 and 8-11, a nucleic acid according to claim 12, a vector according to claim 13 or a cell according to claim 14.

17. A diagnostic comprising a single-chain multiple antigen-binding molecule according to any one of claims 6 or 7.

18. The single-chain multiple antigen-binding molecule according to any one of claims 1-11, a nucleic acid according to claim 12, a vector according to claim 13, or a cell according to claim 14 for use in the therapy, prophylaxis or diagnosis of tumor diseases, autoimmune diseases, inflammatory diseases, disorders of the blood in particular of the coagulation- and/or circulatory-system, disorders of the nervous system and/or infectious diseases.

## Revendications

1. Molécule monocaténaire, à liaison multiple d'antigène, contenant les composants suivants:
(a) un domaine variable d'une chaîne lourde d'une immunoglobuline (VH) ayant une première spécificité (A), et des parties fonctionnelles de celui-ci,
(b) un domaine variable d'une chaîne légère d'une immunoglobuline (VL) ayant une deuxième spécificité (B), et des parties fonctionnelles de celui-ci,
(c) un domaine variable d'une chaîne lourde d'une immunoglobuline (VH) ayant la spécificité (B), et des parties fonctionnelles de celui-ci, ainsi que
(d) un domaine variable d'une chaîne légère d'une immunoglobuline (VL) ayant la spécificité (A), et des parties fonctionnelles de celui-ci,
tandis que les domaines VH et VL sont reliés sous la forme d'une construction VH-VL ou d'une construction VL-VH, **caractérisée en ce que** les deux constructions VH-VL ou les deux constructions VL-VH sont reliées via un peptide (P), et que les constructions VH-VL ou les constructions VL-VH sont reliées via des domaines ayant la même spécificité, et tandis que les spécificités particulières (A) et (B) sont essentiellement identiques à la structure moléculaire d'un domaine d'immunoglobuline de la spécificité indiquée.

2. Molécule monocaténaire à liaison multiple d'antigène selon la revendication 1, **caractérisée en ce que** les domaines VH et VL sont reliés via un lieur peptidique (L) sous la forme d'une construction VH-L-VL ou d'une construction VL-L-VH.

3. Molécule monocaténaire à liaison multiple d'antigène selon la revendication 2, **caractérisée en ce que** le lieur (L) a une longueur d'environ 1-20 acides aminés, de préférence d'environ 1-5 acides aminés.

4. Molécule monocaténaire à liaison multiple d'antigène selon l'une des revendications 1-3, **caractérisée en ce que** le peptide (P) a une longueur d'environ 12-40 acides aminés, de préférence d'environ 12-20 acides aminés, en particulier d'environ 14 acides aminés.

5. Molécule monocaténaire à liaison multiple d'antigène selon l'une des revendications 1-4, **caractérisée en ce que** ladite molécule comprend à titre de composant additionnel un ou plusieurs effecteur(s) (E).

6. Molécule monocaténaire à liaison multiple d'antigène selon l'une des revendications 1-5, **caractérisée en ce que** la première spécificité (A) est dirigée contre une molécule à analyser, et la deuxième spécificité (B) est dirigée contre un analyte.

7. Molécule monocaténaire à liaison multiple d'antigène selon la revendication 6, **caractérisée en ce que** la première spécificité (A) est dirigée contre une molécule à analyser, la deuxième spécificité (B) est dirigée contre une autre molécule à analyser, et l'effecteur (E) est un analyte.

8. Molécule monocaténaire à liaison multiple d'antigène selon l'une des revendications 1-7, **caractérisée en ce que** la première spécificité (A) est dirigée contre une cellule cible, et la deuxième spécificité (B) est dirigée contre un vecteur.

9. Molécule monocaténaire à liaison multiple d'antigène selon la revendication 8, **caractérisée en ce que** le vecteur est un acide nucléique, un peptide ou une protéine cationique, un lipide cationique, un polymère cationique, une porphyrine cationique ou un vecteur viral choisi dans le groupe consistant en un vecteur de AdV, de AAV, de la vaccine, de RSV, de HSV, de la grippe ou de lentivirus.

10. Molécule monocaténaire à liaison multiple d'antigène selon l'une des revendications 1-5, **caractérisée en ce que** la première spécificité (A) est dirigée contre une membrane cellulaire, en particulier contre des lymphocytes, des macrophages, des monocytes, des granulocytes, des cellules hématopoïétiques, des cellules endothéliales, des cellules de muscle lisse, des cellules de muscle strié, des cellules épithéliales, des cellules hépatiques, des cellules rénales, des cellules gliales, des cellules du tissu de soutien, des cellules tumorales ou des cellules de leucémie, ou contre des protéines de la matrice extracellulaire, du système de complément, du système de coagulation, du système de kinine, du plasma sanguin, du tissu de soutien, ou contre les cytokines ou les chimiokines, ou contre les toxines endogènes ou exogènes, ou contre des substances pharmaceutiques, en particulier digitalis, et/ ou contre des agents infectieux tels qu'en particulier les agents infectieux bactériens, viraux et/ou parasitaires.

11. Molécule monocaténaire à liaison multiple d'antigène selon l'une des revendications 1-5 et 10, **caractérisée en ce que** la deuxième spécificité (B) est dirigée contre une membrane cellulaire, en particulier contre les lymphocytes, les macrophages, les monocytes, ou les granulocytes, contre les cytokines, les chimiokines ou les facteurs de croissance, contre les protéines du système de complément, contre les protéines du système de coagulation, contre les protéines fibrinolytiques, contre les enzymes qui sont capables de convertir le précurseur inactif d'une substance active en une substance active, en particulier une substance active cytotoxique, sur la structure cible, contre les hormones peptidiques ou les hormones stéroïdiennes, contre la partie constante d'une immunoglobuline, contre un médiateur, en particulier l'histamine, la sérotonine, le leucotriène, la prostacycline ou la kinine, contre les agents infectieux, contre les toxines endogènes ou exogènes, contre les substances pharmaceutiques, en particulier digitalis.

12. Acide nucléique codant pour une molécule monocaténaire à liaison multiple d'antigène selon l'une des revendications 1-11.

13. Vecteur comprenant un acide nucléique selon la revendication 12.

14. Cellule contenant un acide nucléique selon la revendication 12 ou un vecteur selon la revendication 13.

15. Procédé pour la production d'une molécule monocaténaire à liaison multiple d'antigène, **caractérisé en ce qu'**une cellule selon la revendication 14 est cultivée et le produit d'expression est isolé lorsque cela est approprié.

16. Produit pharmaceutique comprenant une molécule monocaténaire à liaison multiple d'antigène selon l'une des revendications 1-5 et 8-11, un acide nucléique selon la revendication 12, un vecteur selon la revendication 13 ou une cellule selon la revendication 14.

17. Produit de diagnostic comprenant une molécule monocaténaire à liaison multiple d'antigène selon l'une des revendications 6 ou 7.

18. Molécule monocaténaire à liaison multiple d'antigène selon l'une des revendications 1-11, acide nucléique selon la revendication 12, vecteur selon la revendication 13 ou cellule selon la revendication 14 pour utilisation en thérapie, prophylaxie ou diagnostic d'affections tumorales, d'affections auto-immunes, de maladies inflammatoires, de maladies du sang, en particulier du système de coagulation sanguine et/ou du système de circulation sanguine, d'affections du système nerveux et/ ou de maladies infectieuses.


### SEQUENCE LISTING

<110> Affitech AS
<120> Einzelkettiges, mehrfach-antigenbindendes Molekül, dessen Herstellung und Verwendung
<130> 519-5 EPT1
<140> EP
   <141> 1999-04-08
<150> DE19816141.7
   <151> 1998-04-09
<150> DE19827239.1
   <151> 1998-06-18
<150> EP 99106176.3
   <151> 1999-04-08
<160> 18
<170> PatentIn version 3.5
<210> 1
   <211> 14
   <212> PRT
   <213> künstlich
<220>
   <221> 14 amino acid peptide linker, derived from an artificial sequence
   <222> (1)..(14)
<400> 1
   Gly Gly Gly Gly Ser Gly Gly Arg Ala Ser Gly Gly Gly Ser
   1               5                   10
<210> 2
   <211> 15
   <212> PRT
   <213> künstlich
<220>
   <221> 15 amino acid peptide linker
   <222> (1)..(15)
<400> 2
   Gly Gly Gly Gly Ser Gly Gly Arg Ala Ser Gly Gly Gly Gly Ser
   1               5                   10                  15
<210> 3
   <211> 26
   <212> DNA
   <213> künstlich
<220>
   <221> Myc binding site (E-Box)
   <222> (1)..(26)
<400> 3
   ggaagcagac cacgtggtct gcttcc 26
<210> 4
   <211> 17
   <212> DNA
   <213> künstlich
<220>
   <221> E-Box (Myo D binding site)
   <222> (1)..(17)
<400> 4
   agcaggtgtt gggaggc 17
<210> 5
   <211> 41
   <212> DNA
   <213> künstlich
<220>
   <221> GATA-4 binding site
   <222> (1) .. (41)
<400> 5
   ggccgatggg cagatagagg gggccgatgg gcagatagag g 41
<210> 6
   <211> 26
   <212> DNA
   <213> künstlich
<220>
   <221> Myc binding site E-Box
   <222> (1)..(26)
<400> 6
   ggaagcagac cagctggtct gcttcc 26
<210> 7
   <211> 5
   <212> PRT
   <213> künstlich
<220>
   <221> 5 amino acid peptide linker
   <222> (1)..(5)
<400> 7
   Gly Gly Gly Gly Ser
   1 5
<210> 8
   <211> 4
   <212> PRT
   <213> künstlich
<400> 8
   Gly Gly Gly Ser
   1
<210> 9
   <211> 11
   <212> PRT
   <213> künstlich
<220>
   <221> Epitop 9E10 derived of human myc with an additional C-terminal amino acid
   <222> (1)..(11)
<400> 9
   Glu Gln Lys Leu Ile Ser Glu Glu Asp Leu Asn
   1               5                   10
<210> 10
   <211> 6
   <212> PRT
   <213> künstlich
<220>
   <221> Poly-Histidine Tag for purification using IMAC
   <222> (1) .. (6)
<400> 10
   His His His His His His
   1               5
<210> 11
   <211> 45
   <212> DNA
   <213> künstlich
<220>
   <221> Primer Vkappa-Not for for amplification of the VL fragment
   <222> (1) .. (45)
<400> 11
   ttgttctgcg gccgcccgtt tcagctccag cttggtgcca gcacc 45
<210> 12
   <211> 59
   <212> DNA
   <213> künstlich
<220>
   <221> Primer scDb-AscBack for amplification of the VL fragment
   <222> (1)..(59)
<400> 12
   tgcatgctag ggcgcgcctc gggcggaggt ggctcacagg tgcagctggt gcaatctgg 59
<210> 13
   <211> 60
   <212> DNA
   <213> künstlich
<220>
   <221> Primer scDb-AscForkappa for amplification of the VH fragment
   <222> (1) .. (60)
<400> 13
   gctcggtaag gcgcgcccac cgctgccacc gcctccacct aggacggtca gcttggtccc 60
<210> 14
   <211> 32
   <212> DNA
   <213> künstlich
<220>
   <221> Primer pelB-Metminus for amplification of the vH fragment
   <222> (1)..(32)
<400> 14
   ttactcgcgg cccagccggc cacggcccag gt 32
<210> 15
   <211> 7
   <212> PRT
   <213> künstlich
<220>
   <221> 7 amino acid sequence preceeding the antibody fragments, derived from vector pSecTagA with a amino acid substitution at position 6 (Met -> Thr)
   <222> (1)..(7)
<400> 15
   Ala Ala Gln Pro Ala Thr Ala
   1 5
<210> 16
   <211> 37
   <212> PRT
   <213> künstlich
<220>
   <221> Transmembrane-region of the platelet-derived growth factor receptor (PDGFR)
   <222> (1)..(37)
<400> 16
   Leu Pro Phe Lys Val Val Val Ile Ser Ala Ile Ile Ala Leu Val Val
   1 5 10 15
   Leu Thr Ile Ile Ser Leu Ile Ile Leu Ile Met Leu Trp Gln Lys Lys
   20 25 30
   Pro Arg Tyr Glu Ser
   35
<210> 17
   <211> 11
   <212> PRT
   <213> künstlich
<220>
   <221> nuclear location signal of the SV40 large T antigen
   <222> (1) .. (11)
<400> 17
   Pro Lys Lys Lys Arg Lys Val Gly Gly Gly Thr
   1               5                   10
<210> 18
   <211> 6
   <212> PRT
   <213> Rattus norvegicus
<220>
   <221> ER retention signal
   <222> (1) .. (6)
<400> 18
   Ser Glu Lys Asp Glu Leu
   1               5